# EUROPEAN PATENT SPECIFICATION

(11) **EP 2 022 779 B1**
(45) Date of publication and mention of the grant of the patent: **25.05.2011**
(21) Application number: 08103208.8
(22) Date of filing: 31.03.2008
(51) Int. Cl.: C07C 255/41, C08G 63/00, C08L 67/00, C08K 5/00, C08K 5/03, C08K 5/09

(54) **Alkoxy Polyester Compounds, Compositions and Methods of Use Thereof**
Alkoxy-Polyester-Verbindungen, Zusammensetzungen und Verwendungsverfahren dafür
Composés de polyester d'alkoxy, compositions et procédés d'utilisation associés

(30) Priority: 09.08.2007 US 891273
(43) Date of publication of application: 11.02.2009
(73) Proprietor: HallStar Innovations Corp., Chicago, IL 60606-3919 (US)
(72) Inventor: Bonda, Craig A., Winfield, IL 60190 (US); Pavlovic, Anna, Elmwood Park, IL 60707 (US)
(74) Representative: UEXKÜLL & STOLBERG

(56) References cited:
- WO-A-02/42368
- US-A1- 2006 002 869
- US-B2- 6 962 692

## Description

### FIELD OF THE INVENTION

The invention relates to compounds and methods to increase the photostability of UV-degradable photoactive compounds, including polymers. More particularly, the compounds disclosed herein have a formula (I) and can be combined with a photodegradable UV-absorbing compound to increase that compound's photostability: 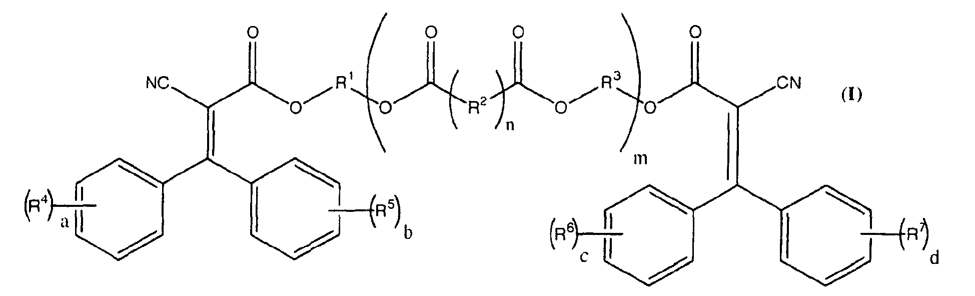 wherein R¹, R², and R³ are the same or different and are selected from the group consisting of C₁-C₃₀ alkyl, C₃-C₈ cycloalkyl, substituted alkyl, substituted cycloalkyl, ester, aryl, heteroaryl, heterocycloalkyl, substituted aryl, substituted heteroaryl, substituted heterocycloalkyl, and amino; R⁴, R⁵, R⁶, and R⁷ are each independently selected from the group consisting of hydrogen and C₁₋₃₀ alkoxy, wherein R⁴ and R⁵ cannot both be hydrogen and R⁶ and R⁷ cannot both be hydrogen; a, b, c, and d are each independently an integer of 0 to 4, preferably 0 or 1; n is an integer of 1 to 100; and m is an integer of 0 to 100. It has been found that a compound of formula (I) quenches the excited state of the chromophore by accepting the excited state energy (*singlet* and sometimes also the *triplet* state), thereby returning the UV-absorbing molecule back to its ground state so that the chromophore can absorb more photons, *e.g.*, from ultraviolet (UV) light, thereby photostabilizing UV-absorbing chromophore-containing organic molecules, particularly avobenzone, octyl methoxy cinnemate (Octinoxate), and octyl salicylate (Octisalate) in photoactive compositions.

### BACKGROUND

The absorption of ultraviolet light by a chromophore-containing organic molecule causes the excitation of an electron in the chromophore moiety from an initially occupied, low energy orbital to a higher energy, previously unoccupied orbital. The energy of the absorbed photon is used to energize an electron and cause it to "jump" to a higher energy orbital, see Turro, Modern Molecular Photochemistry, 1991. Two excited electronic states derive from the electronic orbital configuration produced by UV light absorption. In one state, the electron spins are paired (antiparallel) and in the other state the electron spins are unpaired (parallel). The state with paired spins has no resultant spin magnetic moment, but the state with unpaired spins possesses a net spin magnetic moment. A state with paired spins remains a single state in the presence of a magnetic field, and is termed a singlet state. A state with unpaired spins interacts with a magnetic field and splits into three quantized states, and is termed a triplet state.

In the electronically excited state, the chromophore-containing organic molecule is prone to degrade via a number of known pathways and, therefore, can absorb little or no additional UV light. To photostabilize an electronically excited chromophore-containing organic molecule in order to provide sufficient UV protection, it must be returned to the ground state. There are known photostabilizing sunscreen additives, such as octocrylene and the esters or polyesters of naphthalene dicarboxylic acid of this assignee's U.S. Patent Nos. 6,113,931; 6,284,916; 6,518,451; and 6,551,605, that are capable of quenching excited triplet state energy. Surprisingly, it has been found that alkoxy crylenes, particularly methoxy crylenes, return chromophore-containing organic molecules, particularly avobenzone, octyl methoxycinnamate (Octinoxate), and octyl salicylate (Octisalate), from both an electronically excited singlet state and excited triplet state back to their ground state, thereby photostabilizing the UV-absorbing organic molecules.

Deflandre U.S. 5,576,354 generally discloses a cosmetic sunscreen composition containing at least 1% by weight of an α-cyano-β, β-diphenylacrylate that will photostabilize a dibenzoylmethane derivative, e.g., Parsol 1789 (avobenzone), so long as the composition contains a fatty phase, e.g., glycerol stearates, isopropyl myristate or the like, and so long as the mole ratio of the α-cyano-β, β-diphenylacrylate to the dibenzoylmethane derivative is at least 0.8. The compounds preferred in the '354 patent and disclosed in the examples are octocrylene, which contains no alkoxy radical(s) (UVINULN 539); β, β-bis (4-methoxyphenyl) acrylates (containing no cyano radical); and the α-cyano-β, β-diphenylacrylates, which contain no alkoxy radical(s).

The assignee's U.S. Patent Nos. 7,235,587, 6,919,473, 6,962,692 and 6,800,274 disclose diesters and polyesters that include crylene moieties for photostabilizing photodegradable UV-absorbing compounds. These patents, however, do not disclose diesters or polyesters containing one or more alkoxy crylene moieties.

As stated in this assignee's pending applications, Serial Nos. 10/241,388; 10/361,223; and 10/7865,793, an α-cyano-β,β-diphenylacrylate compound (e.g., octocrylene) is known to quench (accept) the excited triplet state energy of an excited photoactive compound by dissipating the energy kinetically in the form of rapid isomerizations. This process is shown below: 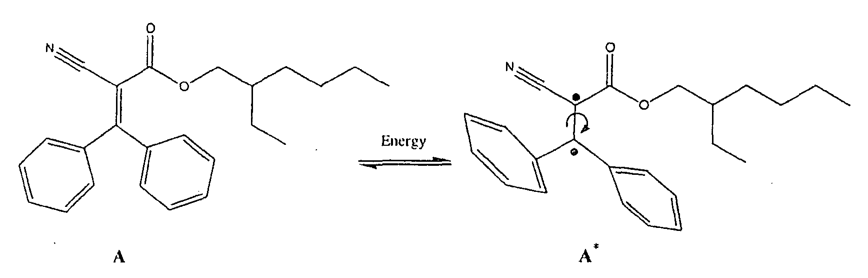 wherein the α-cyano-β,β-diphenylacrylate compound (octocrylene shown above as structure A), accepts the triplet excited state energy from a photoactive compound and forms a diradical (shown above as structure A*) at the α and β positions of the acrylate, which converts the double bond into a single bond and allows for the free rotation of the phenyl groups. This rotation occurs rapidly and efficiently to dissipate any excited triplet state energy accepted by the α-cyano-β,β-diphenylacrylate compound from the photoactive compound.

While octocrylene is able to quench (accept) the triplet excited state energy from a photoactive compound, thereby photostabilizing, to some degree, dibenzoylmethane derivatives, as shown in examples 1, 4, 6 and 8 of Deflandre et al. U.S. Patent No. 5,576,354, there exists a need in the photoactive composition art to find one or more compounds that quench (accept) the singlet excited state energy and preferably also the triplet excited state energy from photoactive compounds.

Quite surprisingly, it has been found that the alkoxy substituted α-cyano-β, β-diphenylacrylate polyesters of formula (I) will quench the electronically excited singlet state energy of UV-absorbing organic molecules, such as the dibenzoylmethane derivatives of U.S. Patent No. 5,576,354, even at low loadings compared to the quantity of UV-absorbing compounds.

### SUMMARY

The present invention is directed to compounds of formula (I) and use of these compounds to increase photostability of a photodegrable compounds and photodegradable polymers.

Thus, one aspect of the invention provides a compound of formula (1): 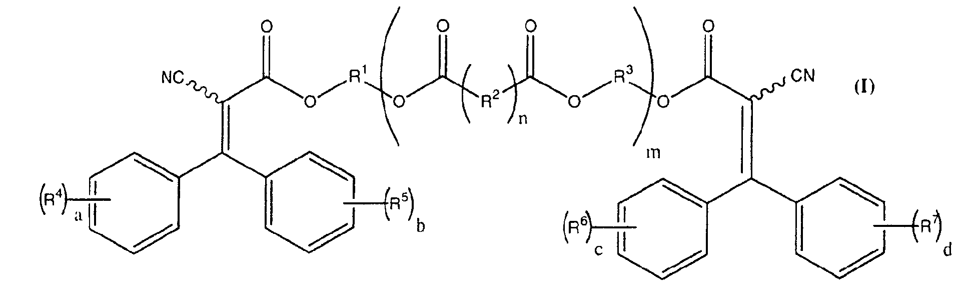 wherein R¹, R², and R³ are the same or different and are selected from the group consisting of C₁-C₃₀ alkyl, C₃-C₈ cycloalkyl, substituted alkyl, substituted cycloalkyl, ester, aryl, heteroaryl, heterocycloalkyl, substituted aryl, substituted heteroaryl, substituted heterocycloalkyl, and amino; R⁴, R⁵, R⁶, and R⁷ are each independently selected from the group consisting of hydrogen and C₁-C₃₀ alkoxy, wherein R⁴ and R⁵ cannot both be hydrogen and R⁶ and R⁷ cannot both be hydrogen; a, b, c, and d are each independently an integer of 0 or 1; n is an integer of 1 to 100; and m is an integer of 0 to 100. The squiggly line of formula (I) indicates that the cyano group can be cis to either of the phenyl rings. In specific embodiments, R⁴, R⁵, R⁶, and R⁷are each independently selected from the group consisting of hydrogen and C₁-C₁₀ alkoxy, and in more specific embodiments, R⁴, R⁵, R⁶, and R⁷ are each independently selected from the group consisting of hydrogen and methoxy. In a specific class of embodiments, a, b, c, and d are each 1 and one of R⁴or R⁶ is methoxy and the other is hydrogen and one of R⁶ and R⁷ is methoxy and the other is hydrogen, wherein the methoxy is in the para position of the phenyl ring. In a specific embodiments, the compound of formula (I) is formula (II): 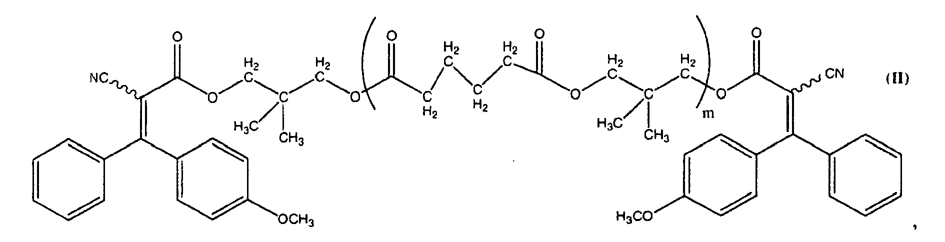 where m is 4 to 6.

Another aspect provides a method of decreasing the photodegradation of a UV-absorbing compound or photodegradable polymer by the addition thereto of an effective amount, e.g., 0.05% to 25%, based on the weight of the photodegradable UV-absorbing compound or photodegradable polymer, preferably 0.1 to 10%, of a compound of formula (1).

Yet another aspect of the invention provides a method for photostabilizing a photodegradable UV-absorbing compound or photodegradable UV-absorbing compound or photodegradable polymer that does not include a UV light-photodegradable photoactive compound, such as a dibenzoylmethane derivative, by the addition of compound of formula (I).

Still another aspect of the invention provides a method for photostabilizing a polymer composition that does not include an additional photodegradable UV-absorbing compound, particularly 4-(1,1-dimethylethyl)-4'-methoxydibenzoylmethane (PARSOLO® 1789), by the addition thereto of a compound of formula (I).

### BRIEF DESCRIPTION OF THE DRAWINGS

Figure 1 is a graph showing the UV stability of a composition having 5% of a compound of formula (II), 5% octyl methoxycinnamate, 2% avobenzone, and 1.8% octocrylene before and after irradiation with 35 minimal erythemal dose (MED) units, wherein 1 MED is 21 millijoules per square centimeter (mJ/cm²); and

Figure 2 is graph showing the UV stability of a composition having 5% of a compound of formula (II), 5% octyl methoxycinnamate, 3% avobenzone, 5% octisalate, 5% homosalate, 4% oxybenzone, and 2.75% octocrylene, before and after irradiation with 35 MED units.

### DEFINITIONS

The term "alkyl" as used herein refers to straight- and branched-chain hydrocarbon groups, preferably containing one to thirty carbon atoms. Examples of alkyl groups are C₁-C₄ alkyl groups. As used herein the designation Cₓ-C_{y}, wherein x and y are integers, denotes a group having from x to y carbon atoms, e.g., a C₁-C₄ alkyl group is an alkyl group having one to four carbon atoms. Nonlimiting examples of alkyl groups include, methyl, ethyl, n-propyl, isopropyl, n-butyl, sec-butyl (2-methylpropyl), and t-butyl (1.1-dimethylethyl).

The term "cycloalkyl" as used herein refers to an aliphatic cyclic hydrocarbon group, preferably containing three to eight carbon atoms. Nonlimiting examples of cycloalkyl groups include cyclopropyl, cyclobutyl, cyclopentyl, and cyclohexyl.

The terms "substituted alkyl" and "substituted cycloalkyl" as used herein refer to an alkyl or cycloalkyl groups having one or more substituents. The substituents can include, but are not limited to, cycloalkyl, aryl, heteroaryl, heterocycloalkyl, substituted aryl, substituted heteroaryl, and substituted heterocycloalkyl. The preferred substituted alkyl groups have one to twenty carbon atoms, not including carbon atoms of the substituent group. Preferably, a substituted alkyl group is mono- or di-substituted at one, two, or three carbon atoms. The substituents can be bound to the same carbon or different carbon atoms.

The term "aryl" as used herein refers to monocyclic, fused bicyclic, and fused tricyclic carbocyclic aromatic ring systems including, but not limited to, phenyl, naphthyl, tetrahydronaphthyl, phenanthrenyl, biphenylenyl, indanyl, indenyl, anthracenyl, and fluorenyl.

The term "heteroaryl" as used herein refers to monocyclic, fused bicyclic, and fused tricyclic aromatic ring systems, wherein one to four-ring atoms are selected from the group consisting of oxygen, nitrogen, and sulfur, and the remaining ring atoms are carbon, said ring system being joined to the remainder of the molecule by any of the ring atoms. Nonlimiting examples of heteroaryl groups include, but are not limited to, pyridyl, pyrazinyl, pyrimidinyl, pyrrolyl, pyrazolyl, imidazolyl, thiazolyl, tetrazolyl, oxazolyl, isooxazolyl, thiadiazolyl, oxadiazolyl, thiophenyl, furanyl, quinolinyl, isoquinolinyl, benzoxazolyl, benzimidazolyl, and benzothiazolyl. Aryl and heteroaryl groups optionally can be substituted with various substitutents. Examples of contemplated substituents include, but are not limited to, halo, OR, N(R)₂, C(=O)N(R)₂, CN, alkyl, substituted alkyl, aryl, substituted aryl, heteroaryl, substituted heteroaryl, cycloalkyl, substituted cycloalkyl, heterocycloalkyl, substituted heterocycloalkyl, O(CH₂)₁₋₃N(R)₂, O(CH₂)₁₋₃CO₂H, and trifluoromethyl.

The term "heterocycloalkyl" as used herein refers to an aliphatic, partially unsaturated or fully saturated, 3- to 14-membered ring system, including single rings of 3 to 8 atoms and bi- and tricyclic ring systems. The heterocycloalkyl ring systems include one to four heteroatoms independently selected from oxygen, nitrogen, and sulfur, wherein a nitrogen and sulfur heteroatom optionally can be oxidized and a nitrogen heteroatom optionally can be substituted. Representative heterocycloalkyl groups include, but are not limited to, pyrrolidinyl, pyrazolinyl, pyrazolidinyl, imidazolinyl, imidazolidinyl, piperidinyl, piperazinyl, oxazolidinyl, isoxazolidinyl, morpholinyl, thiazolidinyl, isothiazolidinyl, and tetrahydrofuryl.

The term "amino" as used herein refers an -NH₂ or -NH- group, wherein each hydrogen in each formula can be replaced with an alkyl, cycloalkyl, aryl, heteroaryl, heterocycloalkyl, substituted alkyl, substituted cycloalkyl, substituted aryl, substituted heteroaryl, or substituted heterocycloalkyl group, i.e., N(R)₂. In the case of -NH₂, the hydrogen atoms also can be replaced with substituents taken together to form a 5- or 6-membered aromatic or non-aromatic ring, wherein one or two carbons of the ring optionally are replaced with a heteroatom selected from the group consisting of sulfur, oxygen, and nitrogen. The ring also optionally can be substituted with an alkyl group. Examples of rings formed by substituents taken together with the nitrogen atom include morpholinyl, phenylpiperazinyl, imidazolyl, pyrrolidinyl, (N-methyl)piperazinyl, and piperidinyl.

The term "substituted diphenylmethylene" as used herein refers to a compound of the general formula: 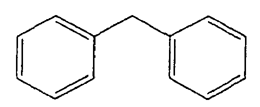 wherein the compound is substituted by a replacement of one, two, or three of the hydrogen atoms resident on each aromatic ring with a substitute selected from the group consisting of halo, OR, N(R)₂, C(=O)N(R)₂, CN, alkyl, substituted alkyl, aryl, substituted aryl, heteroaryl, substituted heteroaryl, cycloalkyl, substituted cycloalkyl, heterocycloalkyl, substituted heterocycloalkyl, O(CH₂)₁₋₃N(R)₂, O(CH2)₁₋₃CO=H, and trifluoromethyl, wherein R=C₁-C₃₀ straight chain or branched.

### DETAILED DESCRIPTION

Sunscreen compositions containing one or more of a photoactive compound, such as a dibenzoylmethane derivative UV-A filter compound, and a derivative of methoxy crylene are described herein. One aspect of the sunscreen compositions described herein are methods of photostabilizing a sunscreen composition including a dibenzoylmethane derivative, such as 4-(1,1-dimethylethyl)-4'-methoxydibenzoylmethane (PARSOL® 1789), wherein one or more photoactive compounds present in a sunscreen composition (e.g., avobenzone) are made more photostable by the addition of a compound of formula (I). Also disclosed herein are methods for filtering out ultra-violet light from human skin including the step of applying a compound of formula (I) to the skin.

Thus, disclosed herein are compounds of formula (I): 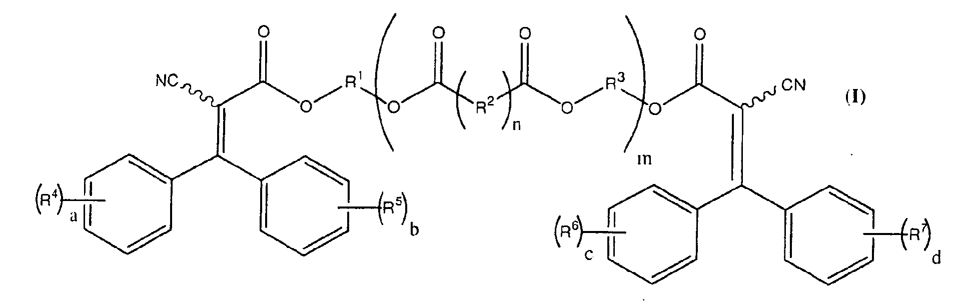 wherein R¹, R², and R³ are the same or different and are selected from the group consisting of C₁-C₃₀ alkyl, C₃-C₈ cycloalkyl, substituted alkyl, substituted cycloalkyl, ester, aryl, heteroaryl, heterocycloalkyl, substituted aryl, substituted heteroaryl, substituted heterocycloalkyl, and amino; R⁴, R⁵, R⁶, and R⁷are each independently selected from the group consisting of hydrogen and C₁-C₃₀ alkoxy, wherein R⁴and R⁵ cannot both be hydrogen and R⁶ and R⁷ cannot both be hydrogen; a, b, c, and d are each independently an integer of 0 or 1; n is an integer of 1 to 100; and m is an integer of 0 to 100. Some specific classes of compounds of formula (1) are the following: (1) R⁴ and R⁷ are each hydrogen, and R⁵ and R⁶ are each alkoxy in the para position and (2) R⁴, R⁵ and R⁷ are each hydrogen, and R⁶ is alkoxy in the para position. In specific cases, the alkoxy is methoxy. In certain cases, R² is butyl (-CH₂CH₂CH₂CH₂-) and R¹ and R³ are each neo-pentyl (-CH₂C(CH₃)₂CH₂-). In specific cases, the compound of formula (I) is Polyester-8 modified with a paramethoxy group at one of the phenyl groups at each terminal diphenyl acrylate moiety (Polyester-8, CAS # 862993-96-2, trade name POLYCRYLENE®).

One contemplated compound of formula (I) is formula (II): 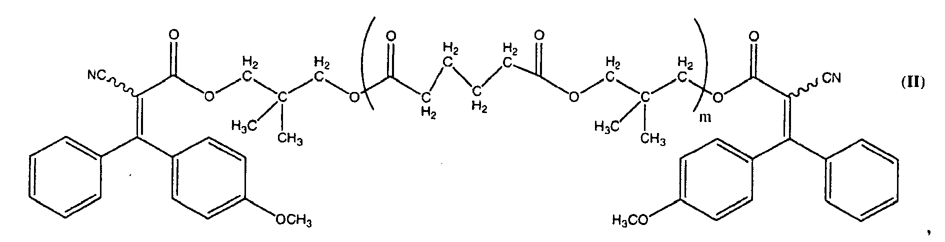 where m is 4 to 8, and the compound of formula (II) has an average molecular weight of about 1900 Da, and a range of molecular weights of about 500 to about 5000 Da, where less than 5% of the molecules has a molecular weight less than 500 Da and less than 25% of the molecules has a molecular weight less than 1000 Da.

A photoactive compound can be considered stable when, for example, after 30 MED irradiation the photoactive compound has retained at least about 90% of its original absorbance at a wavelength or a range of wavelengths of interest (e.g., the wavelength at which or near a photoactive compound has a peak absorbance, such as 350-370 nm for avobenzone). Likewise, a sunscreen composition can include a plurality of photoactive compounds and a sunscreen composition, as a whole, can be considered stable when, for example, after 30 MED irradiation the sunscreen composition has retained at least about 90% of its original absorbance at one or more wavelengths of interest (e.g., at or near the peak absorbance wavelength of the primary photoactive compounds). The alkoxy polyesters of formula (I) described herein are useful photostabilizers and/or photoactive compounds when combined with any single or combination of photoactive compounds identified in Shaath, Nadim, Encyclopedia of UV filters, © 2007, hereby incorporated by reference.

It has surprisingly been found that the addition of one or more of a compound of formula (I) can significantly increase the photostability of the sunscreen composition and/or photounstable components present therein. Without intending to be limited to any particular mechanism of achieving this increase in stability, it is believed that a compound of formula (I) stabilizes a photodegradable UV-absorbing compound, such as a dibenzoylmethane derivative by accepting the electronic singlet excited state energy and sometimes also the triplet excited state energy of the photodegradable UV-absorbing compound once the photodegradable UV-absorbing compound has reached its singlet excited state as a result of the absorption of ultra-violet light. Once a photodegradable UV-absorbing compound is excited, it is prone to degrade according to a number of pathways; however, the degradation of the photodegradable UV-absorbing compound can be substantially reduced or prevented by the use of a compound of formula (I) to quench (accept) the singlet or singlet and triplet excited state energy present in an excited molecule. Thus, in one pathway of degradation, a photodegradable UV-absorbing compound is excited to its singlet state, then proceeds by way of intersystem crossing to the triplet excited state, from which it undergoes a photochemical reaction, thereby preventing the photodegradable UV-absorbing compound from further absorbing ultra-violet radiation. A compound of formula (I) stabilizes a photodegradable UV-absorbing compound by accepting the singlet or singlet and triplet excited state energy of the excited photodegradable UV-absorbing compound in such a way as to convert the excited photodegradable UV-absorbing compound back to a ground state that is capable of reaccepting ultra-violet radiation (energy transfer).

For this process to work continuously, the compound of formula (I) must transfer or convert the energy that was accepted from the excited photodegradable UV-absorbing compound. Without intending to be limited to a particular mechanism, it is believed that when a compound of formula (I) is excited to its singlet and/or triplet state by accepting the singlet state excited energy from a photodegradable UV-absorbing compound or polymer, it dissipates the singlet excited state energy kinetically through a non-destructive isomeriazation of its two phenyl rings. It has been found, quite surprisingly, that by the addition of a compound of formula (I), such a compound is able to accept singlet or singlet and triplet excited state energy from an excited and photounstable UV-absorbing compound or polymer. Thus, according to one possible mechanism, the efficiency of the dissipation of the excited state energy in an excited compound or polymer is greatly improved by a transfer of energy from a singlet excited state compound or polymer to a compound of formula (I), and the dissipation of that energy by the compound of formula (I) so that it can continue to accept the singlet or singlet and triplet state excited energy from the photounstable UV-absorbing compound or polymer.

Thus, the sunscreen compositions disclosed herein include a compound of formula (I). A sunscreen composition disclosed herein can be combined into a cosmetically acceptable carrier, optionally including emollients, stabilizers, emulsifiers, such as those known in the art, and combinations thereof. These additives can be used in preparing an emulsion from an aqueous system and a mixture of a filter system that includes one or more photoactive compounds and a solvent system that includes one or more organic solvents. When made, preferably the emulsion is an oil-in-water emulsion, wherein the oil phase is primarily formed from a mixture of the filter system and solvent system.

A typical sunscreen composition includes one or more photoactive compounds, wherein a photoactive compound acts to absorb UV radiation and thereby protect the substrate (e.g., human skin) from the harmful effects of UV radiation. The absorption process causes a photoactive compound to reach an excited state, wherein the excited state is characterized by the presence of excited energy (e.g., singlet energy or triplet energy), as compared to the ground state of the photoactive compound. Once a photoactive compound reaches an excited state there exists a number of pathways by which the excited photoactive compound can dissipate its excess energy (e.g., singlet or singlet and triplet state energy), however, some of those pathways adversely affect the ability of the photoactive compound to further absorb UV radiation.

A photoactive compound is one that responds to light photoelectrically. In the compositions disclosed herein, a photoactive compound is one that responds to UV radiation photoelectrically. For example, photoactive compounds that respond to UV radiation photoelectrically by rapid photodegradation can benefit highly from the compositions and methods disclosed herein, even though the benefits of the compositions and methods disclosed herein are not limited to such compounds. Photostability is a potential problem with all UV filters because they are deliberately selected as UV-absorbing molecules. In other applications, a photoactive compound may be a pigment or a dye (e.g., a hydrophobic dye).

It is theorized that the following UV filters are photostabilized by the compounds of formula (I), including all of the following, including combinations of any two or more, and include compounds selected from the following categories (with specific examples) including: p-aminobenzoic acid, its salts and its derivatives (ethyl, isobutyl, glyceryl esters; p-dimethylaminobenzoic acid); anthranilates (o-aminobenzoates; methyl, menthyl, phenyl, benzyl, phenylethyl, linalyl, terpinyl, and cyclohexenyl esters); salicylates (octyl, amyl, phenyl, benzyl, menthyl (homosalate), glyceryl, and dipropyleneglycol esters); cinnamic acid derivatives (menthyl and benzyl esters, alpha-phenyl cinnamonitrile; butyl cinnamoyl pyruvate); ferulic acid and its derivatives; dihydroxycinnamic acid derivatives (umbelliferone, methylumbelliferone, methylaceto-umbelliferone); camphor derivatives (3 benzylidene, 4 methylbenzylidene, polyacrylamidomethyl benzylidene, benzalkonium methosulfate, benzylidene camphor sulfonic acid, and terephthalylidene dicamphor sulfonic acid); trihydroxycinnamic acid derivatives (esculetin, methylesculetin, daphnetin, and the glucosides, esculin and daphnin); hydrocarbons (diphenylbutadiene, stilbene); dibenzalacetone; benzalacetophenone; naphtholsulfonates (sodium salts of 2-naphthol-3,6-disulfonic and of 2-naphthol-6,8-disulfonic acids); dihydroxy-naphthoic acid and its salts; o-and p-hydroxy diphenyldisulfonates; coumarin derivatives (7-hydroxy, 7-methyl, 3-phenyl); diazoles (2-acetyl-3-bromoindazole, phenyl benzoxazole, methyl naphthoxazole, various aryl benzothiazoles); quinine salts (bisulfate, sulfate, chloride, oleate, and tannate); quinoline derivatives (8-hydroxyquinoline salts, 2-phenylquinoline); hydroxy- or methoxy-substituted benzophenones; uric acid derivatives; vilouric acid derivatives; tannic acid and its derivatives; hydroquinone; and benzophenones (oxybenzone, sulisobenzone, dioxybenzone, benzoresorcinol, 2,2',4,4'-tetrahydroxybenzophenone, 2,2'-dihydroxy-4,4'-dimethoxybenzophenone, octabenzone, 4-isopropyldibenzoylmethane, butylmethoxydibenzoylmethane, etocrylene, and 4-isopropyl-dibenzoylmethane),

The following UV-filters should be particularly photostabilized by the compounds of formula (I): 2-ethylhexyl p-methoxycinnamate, isoamyl methoxycinnamate, 4,4'-t-butyl methoxydibenzoylmethane, octyldimethyl p-aminobenzoic acid, ethyl 4-[bis(hydroxypropyl)]aminobenzoate, 2-ethylhexylsalicylate, glycerol p-aminobenzoate, 3,3,5-trimethylcyclohexylsalicylate, methylanthranilate, p-dimethylaminobenzoic acid or aminobenzoate, 2-ethylhexyl p-dimethylaminobenzoate, and combinations thereof.

For a product marketed in the United States, preferred cosmetically acceptable photoactive compounds and concentrations (reported as a percentage by weight of the total cosmetic sunscreen composition) include: aminobenzoic acid (also called para aminobenzoic acid and PABA; 15% or less), avobenzone (also called butyl methoxy dibenzoylmethane; 3% or less), cinoxate (also called 2 ethoxyethyl p methoxycinnamate; 3% or less), dioxybenzone (also called benzophenone 8; 3% or less), homosalate (15% or less), menthyl anthranilate (also called menthyl 2 aminobenzoate; 5% or less), octocrylene (also called 2 ethylhexyl 2 cyano 3,3 diphenylacrylate; 10% or less), octyl methoxycinnamate (7.5% or less), octyl salicylate (also called 2 ethylhexyl salicylate; 5% or less), oxybenzone (also called benzophenone 3; 6% or less), padimate O (also called octyl dimethyl PABA; 8% or less), phenylbenzimidazole sulfonic acid (water soluble; 4% or less), sulisobenzone (also called benzophenone 4; 10% or less), titanium dioxide (25% or less), trolamine salicylate (also called triethanolamine salicylate; 12% or less), and zinc oxide (25% or less).

Other preferred cosmetically acceptable photoactive compounds and preferred concentrations (percent by weight of the total cosmetic sunscreen composition) include diethanolamine methoxycinnamate (10% or less), ethyl-[bis(hydroxypropyl)] aminobenzoate (5% or less), glyceryl aminobenzoate (3% or less), 4 isopropyl dibenzoylmethane (5% or less), 4 methylbenzylidene camphor (6% or less), terephthalylidene dicamphor sulfonic acid (10% or less), and sulisobenzone (also called benzophenone 4, 10% or less).

For a product marketed in the European Union, preferred cosmetically acceptable photoactive compounds and preferred concentrations (reported as a percentage by weight of the total cosmetic sunscreen composition) include: PABA (5% or less), camphor benzalkonium methosulfate (6% or less), homosalate (10% or less), benzophenone 3 (10% or less), phenylbenzimidazole sulfonic acid (8% or less, expressed as acid), terephthalidene dicamphor sulfonic acid (10% or less, expressed as acid), butyl methoxydibenzoylmethane (5% or less), benzylidene camphor sulfonic acid (6% or less, expressed as acid), octocrylene (10% or less, expressed as acid), polyacrylamidomethyl benzylidene camphor (6% or less), ethylhexyl methoxycinnamate (30% or less), PEG 25 PABA (10% or less), isoamyl p methoxycinnamate (10% or less), ethylhexyl triazone (5% or less), drometrizole trielloxane (15% or less), diethylhexyl butamido triazone (10% or less), 4 methylbenzylidene camphor (4% or less), 3 benzylidene camphor (2% or less), ethylhexyl salicylate (5% or less), ethylhexyl dimethyl PABA (8% or less), benzophenone 4 (5%, expressed as acid), methylene bis benztriazolyl tetramethylbutylphenol (10% or less), disodium phenyl dibenzimidazole tetrasulfonate (10% or less, expressed as acid), bis ethylhexyloxyphenol methoxyphenol triazine (10% or less), methylene bisbenzotriazolyl tetramethylbutylphenol (10% or less, also called TINOSORB M), bisethylhexyloxyphenol methoxyphenyl triazine (10% or less, also called TINOSORB S), and diethylamino hydroxyl benzoyl hexyl benzoate.

All of the above described UV filters are commercially available. For example, suitable commercially available organic UV filters are identified by trade name and supplier in Table I below:

**Table 1**

| **CTFA Name** | **Trade Name** | **Supplier** |
|---|---|---|
| benzophenone-3 | UVINUL M-40 | BASF Chemical Co. |
| benzophenone-4 | UVINUL MS-40 | BASF Chemical Co. |
| benzophenone-8 | SPECTRA-SORB UV-24 | American Cyanamid |
| DEA-methoxycinnamate | BERNEL HYDRO | Bernel Chemical |
| diethylamino hydroxybenzoyl hexyl benzoate | UVINUL A-PLUS | BASF Chemical Co. |
| diethylhexyl butamido triazone | UVISORB HEB | 3V-Sigma |
| disodium phenyl dibenzylimidazole | NEO HELIOPAN AP | Symrise |
| ethyl dihydroxypropyl-PABA | AMERSCREEN P | Amerchol Corp. |
| glyceryl PABA | NIPA G.M.P.A. | Nipa Labs. |
| homosalate | KEMESTER HMS | Humko Chemical |
| menthyl anthranilate | SUNAROME UVA | Felton Worldwide |
| octocrylene | UVINUL N-539 | BASF Chemical Co. |
| octyl dimethyl PABA | AMERSCOL | Amerchol Corp. |
| octyl methoxycinnamate | PARSOL MCX | Bernel Chemical |
| PABA | PABA | National Starch |
| 2-phenylbenzimidazole-5-sulphonic acid | EUSOLEX 6300 | EM Industries |
| TEA salicylate | SUNAROME W | Felton Worldwide |
| 2-(4-methylbenzildene)-camphor | EUSOLEX 6300 | EM Industries |
| benzophenone-1 | UVINUL 400 | BASF Chemical Co. |
| benzophenone-2 | UVINUL D-50 | BASF Chemical Co. |
| benzophenone-6 | UVINUL D-49 | BASF Chemical Co. |
| benzophenone-12 | UVINUL 408 | BASF Chemical Co. |
| 4-isopropyl dibenzoyl methane | EUSOLEX 8020 | EM Industries |
| butyl methoxy dibenzoyl methane | PARSOL 1789 | Givaudan Corp. |
| etocrylene | UVINUL N-35 | BASF Chemical Co. |
| methylene bisbenzotriazolyl tetramethylbutylphenol | TINOSORB M | Ciba Specialty Chemicals |
| bisethylhexyloxyphenol methoxyphenyl triazine. | TINOSORB S | Ciba Specialty Chemicals |

A sunscreen composition disclosed herein can include a variety of photoactive compounds, including one or more UV-A photoactive compounds and one or more UV-B photoactive compounds. Preferably, a sunscreen composition includes a photoactive compound selected from the group consisting of p-aminobenzoic acid and salts and derivatives thereof; anthranilate and derivatives thereof; dibenzoylmethane and derivatives thereof; salicylate and derivatives thereof; cinnamic acid and derivatives thereof; dihydroxycinnamic acid and derivatives thereof; camphor and salts and derivatives thereof; trihydroxycinnamic acid and derivatives thereof; dibenzalacetone naphtholsulfonate and salts and derivatives thereof; benzalacetophenone naphtholsulfonate and salts and derivatives thereof; dihydroxy-naphthoic acid and salts thereof; o-hydroxydiphenyldisulfonate and salts and derivatives thereof; p-hydroxydiphenyldisulfonate and salts and derivatives thereof; coumarin and derivatives thereof; diazole derivatives; quinine derivatives and salts thereof; quinoline derivatives; hydroxy-substituted benzophenone derivatives; methoxy-substituted benzophenone derivatives; uric acid derivatives; vilouric acid derivatives; tannic acid and derivatives thereof; hydroquinone; benzophenone derivatives; 1,3,5-triazine derivatives, phenyldibenzimidazole tetrasulfonate and salts and derivatives thereof; terephthalylidene dicamphor sulfonic acid and salts and derivatives thereof; methylene bis-benzotriazolyl tetramethylbutylphenol and salts and derivatives thereof; bis-ethylhexyloxyphenol methoxyphenyl triazine and salts and derivatives thereof; diethylamino hydroxybenzoyl hexyl benzoate and salts and derivatives thereof; ferulic acid and its derivatives; and combinations of the foregoing.

UV A radiation (about 320 nm to about 400 nm), is recognized as contributing to causing damage, to skin particularly to very lightly colored or sensitive skin. A sunscreen composition disclosed herein preferably includes a UV-A photoactive compound. Preferably, a sunscreen composition disclosed herein includes a dibenzoylmethane derivative UV-A photoactive compound. Preferred dibenzoylmethane derivatives include, 2-methyldibenzoylmethane; 4-methyldibenzoylmethane; 4-isopropyldibenzoylmethane; 4-tert-butyldibenzoylmethane; 2,4-dimethyldibenzoylmethane; 2,5-dimethyldibenzoylmethane; 4,4'-diisopropyldibenzoylmethane; 4,4'-dimethoxydibenzoylmethane: 4-tert-butyl-4'-methoxydibenzoylmethane; 2-methyl-5-isopropyl-4'-methoxydibenzoylmethane; 2-methyl-5-tert-butyl-4'-methoxydibenzoylmethane; 2,4-dimethyl-4'-methoxydibenzoylmethane; 2,6-dimethyl-4-tert-butyl-4'-methoxydibenzoylmethane, and combinations thereof.

A preferred combination of photoactive compounds in a sunscreen composition includes a UV-A and a UV-B photoactive compound. However, when 2-ethylhexyl-p-methoxycinnamate is included in a mixture with a dibenzoylmethane derivative, the dibenzoylmethane derivative can become particularly unstable. Without intending to be limited to any particular mechanism, it is believed that the cinnamate ester reacts with an excited-state dibenzoylmethane derivative in a bimolecular pathway that renders both the dibenzoylmethane derivative and the cinnamate ester incapable of absorbing UV radiation. It has been found, quite surprisingly, that the use of one or more of a compound of formula (I) increases the stability of a sunscreen composition that includes 2-ethylhexyl-p-methoxycinnamate and a dibenzoylmethane derivative by quenching electronic singlet state or singlet and triplet state excited energy from one or both UV-absorbers. Thus, one embodiment of a sunscreen composition includes 2-ethylhexyl-p-methoxycinnamate, a dibenzoylmethane derivative, and one or more of a compound of formula (I).

It is preferred that a compound of formula (I) is present in a sunscreen composition in a range of 0.1% to about 25% by weight of the total weight of the composition, more preferably about 0.1% to about 10%.

### EXAMPLES

The following examples are provided to illustrate the invention but are not intended to limit the scope of the invention.

### Example 1 Preparation of a compounds of formula (I)

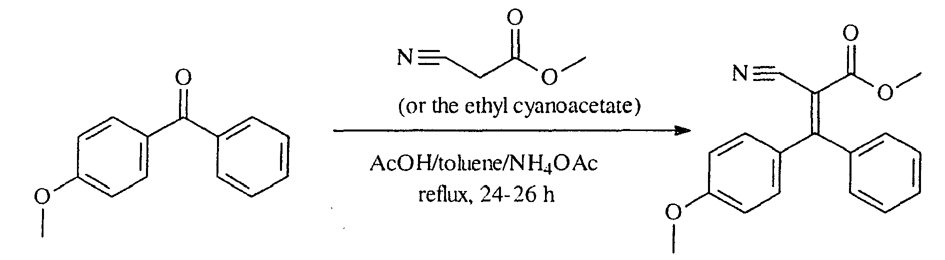

**Synthesis of 1:** 4-Methoxy benzophenone (MW=182.22 g/mole; 500g; 2.74 mole; 1 mole equivalence) and methyl cyanoacetate (MW=99.09 g/mole; 367.06 g; 3.70 mole; 1.35 mole equivalence) were placed in 1-L 3-neck flask assembled with mechanical stirrer and nitrogen inlet, which provided continuous flow of nitrogen through the reaction mixture (nitrogen is bubbled through the reaction mixture). Next, toluene (1200 ml) and acetic acid (240 ml; ratio of toluene/acetic acid = 5/1) are added to the flask followed by ammonium acetate (MW=77.09 g/mole; 21.12g; 0.274 mole, 0.1 mole equivalence; the catalyst is added 4 times this amount during reaction time). The flask is then assembled with Dean-Stark receiver through which reaction water is being continuously removed.

Aliquots of the reaction mixture are taken to check the rate of completion of the reaction. The amount of water expected from this reaction is 49.5 ml. However, 120 ml of water phase was collected. This is due to the fact that the water is distilled in form of a mixture, water/acetic acid/toluene. To prevent losing the methyl acetate from the reaction mixture, it helps to put a short packed column between Dean-Stark receiver and the flask.

The reaction mixture is cooled to room temperature and ethyl acetate is added to dissolve all solids before the crude mixture is washed several time with water, to remove acetic acid and salts. The solvents are then removed from the reaction mixture by distillation. The crude solid product is re-crystallized from hot methanol (or toluene/methanol mixture, if such is preferred).

The finished product begins to crystallize out from the cooling reaction mixture and thus can be filtered off, but it still is very acidic, so the crystals ought to be washed with water/methanol mixture to wash out any acetic acid and salts residues. Thus obtained the product then can be re-crystallized and the mother liquor can be washed with water, dried, and second crop of the product can be obtained.

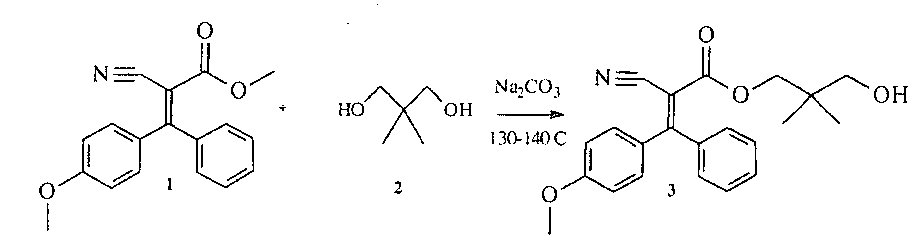

**Synthesis of 3:** Methyl/ethyl 2-cyano-3-(4'-methoxyphenyl)-3-diphenylacrylate (1 mole equivalent) is dissolved in excess of NPG (from 5 to 6 mole equivalent) placed in 3-neck round bottom flask, and sodium carbonate (0.3 mole equivalent) is added. Next, the reaction mixture is continuously heated at 135 °C (optimum temperature may very from 130 °C to 140 °C depending on the type of an ester). Throughout reaction time, methanol/ethanol is removed from the reaction mixture by continuous distillation. When reaction is completed (within one to two hours), toluene is added to prevent solidification of the crude product mixture, and then sodium carbonate is filtered off when the solution is still hot (**Note 1**). The toluene solution is washed three times with water to remove completely the excess of glycol (**Note 2**). Toluene is then removed by distillation and the product is recrystallized from toluene or methanol/toluene mixture (**Note 3**).

**Note 1**: The amount of toluene needed is calculated by estimating volume of the crude product and multiplying that amount by factor 1.5 (Vsolvent = Vcrude product x 1.5). After work-up is completed, the excess of solvent used can be recovered.

**Note 2**: The volume of water in the first wash was equal to volume of the organic solvent. The volume of water of the second and third washes was equal to ¾ of the first volume.

**Note 3**: The highest yield of the product is obtained when NPG is used for the transesterification reaction. Equal amount of toluene (by weight of the product) is added to the molten product. Upon cooling, while stirring is maintained, the product will beautify precipitated in form of a powder. Filter off the product and dry it under vacuum.

**Synthesis of Compound of Formula (I):** Polymer A(NA)ₙA (1 mole equivalence; A stands for adipic acid and N stands for NPG, which is neopentyl glycol; **Note 4**), NPG ester of 2-cyano-3-(4'-methoxyphenyl-3-phenyl-2-propenoic acid, hereinafter referred to as NPG methoxycrylene (1.5 mole equivalence), dibutyl ether (**Note 5**), methane sulfonic acid catalyst (0.3 % of the total batch weight; **Note 6**), and antioxidant (sodium hypophosphite; 0.03% of total batch weight, **Note 7**) were placed in 3-neck round bottom flask. The reaction flask was assembled with mechanical stirrer, thermometer and nitrogen inlet, Dean-Stark adapter, and condenser. The reaction mixture was heated to temperature at which solvent will reflux (**Note 5**) and the reflux was maintained for two hours, continuously removing water. After two hours, 2-octyldodecanol (0.4 mole equivalence, **Note 8**) was added and the reaction was refluxed for additional 2-4 hours. When acid value of the reaction was sufficiently low (less than 10), sodium bicarbonate (0.5 % of the total batch weight) was added to the reaction vessel and the product was stirred for 30 minutes before filtration (**Note 9**). The product was filtered through filtration aid (Celite) at higher temperature (**Note 10**). The solvent was removed after filtration through vacuum distillation.

**Note 4**: Based on acid value of the polymeric backbone A(NA)ₙA, a molecular weight of the polymer was calculated. This molecular weight served as the basis for calculating the needed amount of NPG methoxycrylene and 2-octyldodecanol. 1.5-mole equivalence of the NPG crylene is related to 75 mole % of the stoichiometric amount of the terminator.

**Note 5**: Ideal reflux temperature should fall between 140 and 150 °C. Dibutyl ether, which boils at 141°C, was chosen for the reaction as the preferred solvent. The recommended amount of the solvent was calculated by taking 20 % of the batch weight [A(NA)nA polymer plus NPG methoxycrylene plus 2-octyldodecanol].

**Note 6**: The amount of the catalyst was calculated by multiplying the total batch weight (polymer A(NA)ₙA plus NPG methoxycrylene plus 2-octyldodecanol plus solvent) by 0.3 %. Two thirds of the calculated amount of the catalyst was added in the beginning of the reaction and the remaining amount was added together with 2-octyldodecanol.

**Note 7**: The amount of the antioxidant was calculated in a similar way as the amount of the catalyst (see note 5). The calculated amount of antioxidant was added to the reaction on loading.

**Note 8**: 0.4-mole equivalence means that 20-mole % (about 8 % w/w of finished product) of 2-octyldodecanol was used to terminate the polymer. 2-octyldodecanol is always added after NPG crylene completely reacts with the polymer.

**Note 9**: To remove MSA catalyst sodium bicarbonate (0.5 % of the batch weight) was added before filtration.

**Note 10**: The final product, methoxycrylene-terminated polyester, has rather high viscosity, but it can be efficiently filtered with a Buchner funnel heated to the temperature of steam. The filtration is made easier when the solvents are removed after filtration and not before it.

### Example 2

A series of sunscreen compositions was prepared by mixing the ingredients as shown in Table 2, below. The compound of formula (I) used in the below formulations of all examples was as prepared in Example 1, and the compound has an average molecular weight of about 1900 Da, and a range of molecular weight of about 1500 to about 2500 Da. Also added to the compound of formula (II) was about 5 wt% to about 10 wt%, typically about 8 wt%, of a compound of formula (III) (where m is 4 to 6) to decrease the viscosity of the compound of formula (II) and make it easier to handle.

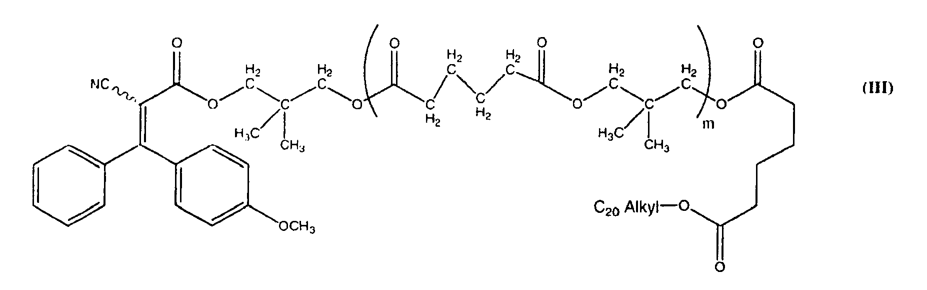

**Table 2**

| **Ingredients** | **Composition** **A** | **Composition** **B** |
|---|---|---|
| **Oil Phase** | | |
| Avobenzone | 2.00% | 2.00% |
| Octyl-p-methoxy Cinnamate | 5.00% | 5.00% |
| Phenethyl Benzoate | 7.50% | 7.50% |
| Benzophenone-3 | 0.49% | 0.49% |
| Octocrylene | 1.80% | 1.80% |
| Compound of formula (I) | 0% | 5.00% |
| Polyisobutene | 7.00% | 0.00% |
| | | |
| **Emulsifiers** | | |
| Acrylates/C10-20 alkyl acrylate crosspolymer | 0.25% | 0.25% |
| Sorbitan laurate 2 | 0.20% | 0.20% |
| | | |
| **Water Phase** | | |
| Disodium EDTA | 0.10% | 0.10% |
| Cetyl hydroxyethylcellulose | 0.30% | 0.30% |
| Glycerin | 4.00% | 4.00% |
| Benzyl alcohol | 1.00% | 1.00% |
| Methylparaben | 0.10% | 0.10% |
| Propylparaben | 0.05% | 0.05% |
| Triethanolamine | 0.40% | 0.40% |
| Water | 69.81% | 71.81% |

A vessel was charged with the water and disodium EDTA. The solution was heated to 85°C. Cetyl hydroxyethyl cellulose was added and the mixture removed from heat and stirred until all the cellulose was dissolved. In a second vessel, all the oil phase ingredients except for the avobenzone were mixed together with the sorbitan laurate. Then, the avobenzone was added and the resulting mixture heated to 45°C. The mixture was stirred until a clear solution was obtained. Once the solution was clear, the acrylates/C10-20 alkyl acrylate crosspolymer was added and stirred to completely incorporate. Next, both the water and oil phases were brought to 45°C and the oil phase was added to the water phase while maintaining the temperature at 45°C and stirring. The resulting mixture was removed from heat and a mixture of glycerin and triethanolamine was added. Agitation was increased as the mixture thickened. Then, a mixture of the remaining ingredients was added. More water was added as necessary and the composition was packaged when the temperature of the mixture was less than 35°C.

The resulting sunscreens were tested for photostability by measuring absorbance on a Labsphere UV 1000S Ultraviolet Transmittance Analyzer (software version 1.27) before and after irradiation with a Solar Light Company model 16S solar simulator with exposure by 35 MED. Output was monitored by a PMA 2105 UV B DCS Detector (biologically weighted) and controlled by a PMA 2100 Automatic Dose Controller (Solar Light Co.). The photostablity measurements of Composition B showed it had a UVA/UVB ratio of 0.88 and an average SPF of 23.83. After exposure to 35 MED, Composition A lost 80.47% of its UVA protection, 52.01% of its UVB protection, and 70.42% of its SPF. Composition B lost 44.51% of its UVA protection, 15.81% of its UVB protection, and 23.41% of its SPF.

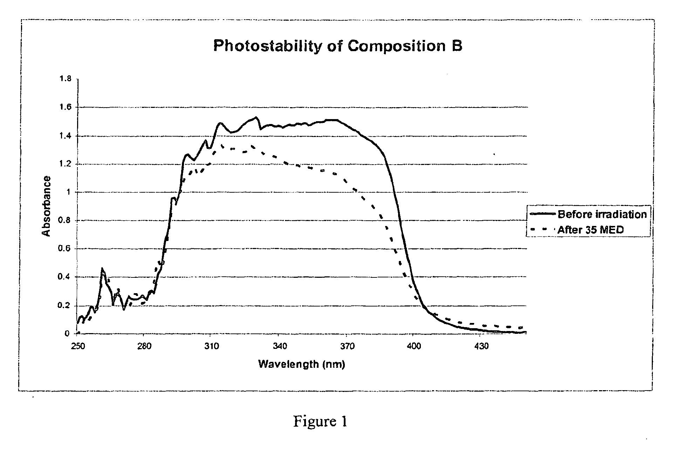

To test stability, a slide was positioned on the UV transmittance analyzer using registration marks, and a scan of a 1 cm spot on the slide was performed. The slide was then transferred to a holder placed adjacent to the solar simulator and, using a calipers, was positioned such that the beam of UV radiation exiting the solar simulator illuminated the same 1 cm spot on the slide. The following software settings were used: UV B = 290-320 nm; UV A = 320-400 nm. Following an exposure of 5 MED, the slide was again placed in position on the UV transmittance analyzer, and a scan of the exposed spot was performed. The procedure was repeated on the same 1 cm spot on the slide until the desired total radiation dosage was achieved. The results of this analysis for Composition B is shown in Figure 1.

### Example 2

Another composition was prepared, as outlined below, with the components as reported in Table 3.

**Table 3**

| **Ingredients** | **CompositionC** |
|---|---|
| **Oil Phase** | |
| Avobenzone | 3.00% |
| Octyl-*p*-methoxy Cinnamate | 5.00% |
| Homosalate | 7.50% |
| Benzophenone-3 | 4.00% |
| Octocrylene | 2.75% |
| Compound of formula (I) | 5.00% |
| VP/Eicosene copolymer | 1.00% |
| Silica | 0.40% |
| | |
| **Emulsifiers** | |
| Acrylates/C10-20 alkyl acrylate crosspolymer | 0.25% |
| Sorbitan laurate 2 | 0.20% |
| | |
| **Water Phase** | |
| Disodium EDTA | 0.10% |
| Cetyl hydroxyethylcellulose | 0.30% |
| Glycerin | 4.00% |
| Benzyl alcohol | 1.00% |
| Methylparaben | 0.10% |
| Propylparaben | 0.05% |
| Triethanolamine | 0.40% |
| Water | 64.95% |

A vessel was charged with the water and disodium EDTA. The solution was heated to 85°C. Cetyl hydroxyethylcellulose was added and the mixture removed from heat and stirred until all the cellulose was dissolved. In a second vessel, all the oil phase ingredients except for the silica and VP/eicosene copolymer were mixed together with the sorbitan laurate. Then, the VP/eicosene copolymer was added and the resulting mixture was stirred until the mixture was a clear solution. Next, the acrylates/C10-20 alkyl acrylate crosspolymer was added to the oil phase solution and stirred until completely incorporated. Next, the silica was added and the mixture was agitated until fully wetted. Next, both the water and oil phases were brought to 45°C and the oil phase was added to the water phase while maintaining the temperature at 45°C and stirring. The resulting mixture was removed from heat and a mixture of glycerin and triethanolamine was added. Agitation was increased as the mixture thickened. Then, a mixture of the remaining ingredients was added. More water was added as necessary and the composition was packaged when the temperature of the mixture was less than 35°C.

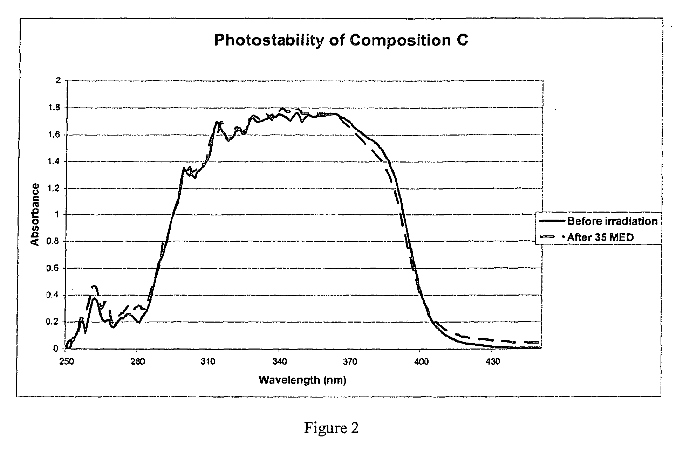

Composition C was also assessed for stability. After exposure to 35 MED, it lost 8.46% of its UVA protection, 1.18% of its UVB protection, and 0.32 of its SPF.
Additionally, its stability is shown in Figure 2.

## Claims

1. A compound of formula (I): 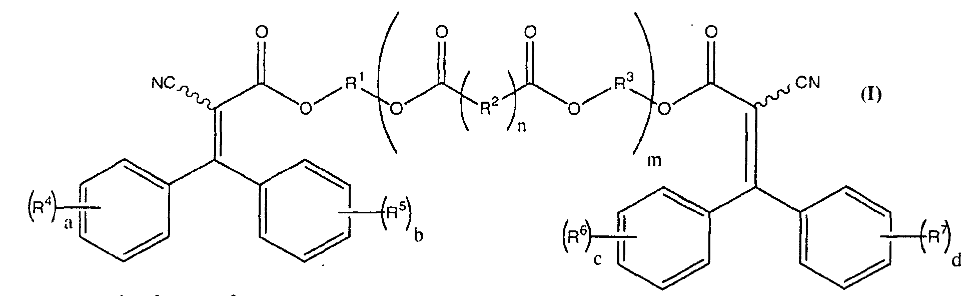 wherein R¹, R², and R³ are the same or different and are selected from the group consisting of C₁-C₃₀ alkyl, C₃-C₈ cycloalkyl, substituted alkyl, substituted cycloalkyl, ester, aryl, heteroaryl, heterocycloalkyl, substituted aryl, substituted heteroaryl, substituted heterocycloalkyl, and amino; R⁴, R⁵, R⁶, and R⁷ are each independently selected from the group consisting of hydrogen and C₁-C₃₀ alkoxy, wherein R⁴ and R⁵ cannot both be hydrogen and R⁶ and R⁷ cannot both be hydrogen; a, b, c, and d are each independently 0 or I; n is an integer of 1 to 100; and m is an integer of 0 to 100.

2. The compound of claim 1, wherein a, b, c, and d are each 1.

3. The compound of claim 1, wherein R⁴, R⁵, R⁶, and R⁷ are each independently selected from the group consisting of hydrogen and C₁-C₈ alkoxy.

4. The compound of claim 3, wherein R⁴, R⁵, R⁶, and R⁷ are each independently selected from the group consisting of hydrogen and methoxy.

5. The compound of claim 4, wherein each methoxy is in the para position.

6. The compound of claim 1, wherein R¹ and R³ are each neopentyl.

7. The compound of claim 1, wherein R² is butyl.

8. The compound of claim 1, having the formula: 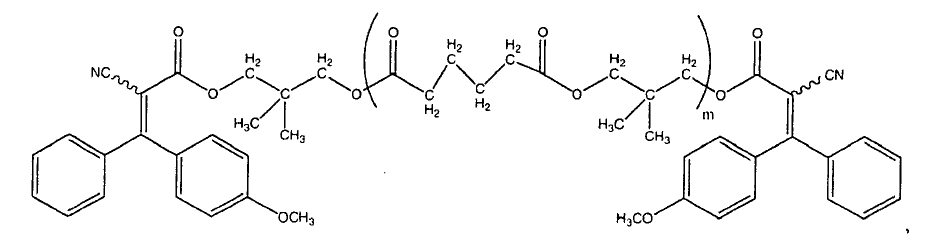 wherein m is an integer of 4 to 8.

9. A composition comprising a compound of claim 1.

10. The composition of claim 9, further comprising an additive selected from the group consisting of cosmetically acceptable emollients, stabilizers, emulsifiers, thickeners, humectants, surfactants, preservatives, vitamins, antifoaming agents, fragrances, anti-irritants, organomodified silicones, chelators, opacifiers, polar oils, nonpolar oils, waxes, alcohols, polyols, propellants, colorants, pigments, and combinations thereof.

11. The composition of claim 9, further comprising a dibenzoylmethane derivative.

12. The composition of claim 11, wherein said dibenzoyl methane derivative is selected from the group consisting of 2-methyldibenzoylmethane; 4-methyldibenzoylmethane; 4-isopropyldibenzoylmethane; 4-tert-butyldibenzoylmethane; 2,4-dimethyldibenzoylmethane; 2,5-dimethyldibenzoylmethane; 4,4'-diisopropyldibenzoylmethane; 4,4'-dimethoxydibenzoylmethane; 4-tert-butyl-4'-methoxydibenzoylmethane; 2-methyl-5-isopropyl-4'-methoxydibenzoylmethane; 2-methyl-5-tert-butyl-4'-methoxydibenzoylmethane; 2,4-dimethyl-4'-methoxydibenzoylmethane; 2,6-dimethyl-4-ter-t-butyl-4'-methoxydibenzoylmethane, and combinations thereof.

13. The composition of claim 9, further comprising a photoactive compound selected from the group consisting of p-aminobenzoic acid and salts and derivatives thereof; anthranilate and derivatives thereof; salicylate and derivatives thereof; cinnamic acid and derivatives thereof; dihydroxycinnamic acid and derivatives thereof; camphor and salts and derivatives thereof; trihydroxycinnamic acid and derivatives thereof; dibenzalacetone naphtholsulfonate and salts and derivatives thereof; benzalacetophenone naphtholsulfonate and salts and derivatives thereof; dihydroxy-naphthoic acid and salts thereof; naphthalene dicarboxylic acids, derivatives, dimers, oligimers, polymers, and salts and combinations thereof; o-hydroxydiphenyldisulfonate and salts and derivatives thereof; p-hydroxydiphenyldisulfonate and salts and derivatives thereof; coumarin and derivatives thereof; diazole derivatives; quinine derivatives and salts thereof; quinoline derivatives; hydroxy-substituted benzophenone derivatives; methoxy-substituted benzophenone derivatives; uric acid derivatives; vilouric acid derivatives; tannic acid and derivatives thereof; hydroquinone; benzophenone derivatives; 1,3,5-triazine derivatives, phenyldibenzimidazole tetrasulfonate and salts and derivatives thereof; terephthalylidene dicamphor sulfonic acid and salts and derivatives thereof; methylene bis-benzotriazolyl tetramethylbutylphenol and salts and derivatives thereof; bis-ethylhexyloxyphenol methoxyphenyl triazine and salts and derivatives thereof; diethylamino hydroxybenzoyl hexyl benzoate and salts and derivatives thereof; and combinations thereof.

14. A method of photostabilizing a photodegradable UV-absorbing compound or polymer comprising adding a photostabilizing amount of a compound of claim 1 to the photodegradable UV-absorbing compound or polymer.

15. The method of claim 14, wherein the photodegradable UV-absorbing compound or polymer is a dibenzoylmethane derivative selected from the group consisting of 2-methyldibenzoylmethane; 4-methyldibenzoylmethane; 4-isopropyldibenzoylmethane; 4-tert-butyldibenzoylmethane; 2,4-dimethyldibenzoylmethane; 2,5-dimethyldibenzoylmethane; 4,4'-diisopropyldibenzoylmethane; 4,4'-dimethoxydibenzoylmethane; 4-tert-butyl-4'-methoxydibenzoylmethane; 2-methyl-5-isopropyl-4'-methoxydibenzoylmethane; 2-methyl-5-tert-butyl-4'-methoxydibenzoylmethane; 2,4-dimethyl-4'-methoxydibenzoylmethane; 2,6-dimethyl-4-tert-butyl-4'-methoxydibenzoylmethane, and combinations thereof.

16. The method of claim 15 further comprising adding a triplet quencher selected from the group consisting of octocrylene, methyl benzylidene camphor, diethylhexyl 2,6-naphthalate, diethylhexyl syringylidene malonate, and combinations thereof.

17. The method of claim 15 further comprising adding benzophenone-3.

18. The method of claim 15 further comprising adding octyl salicylate.

19. The method of claim 15 further comprising adding a cinnamate ester.

20. The method of claim 19, wherein the cinnamate ester is 2-ethylhexyl-p-methoxycinnemate.

21. The method of claim 19 further comprising adding bis-ethylhexyloxyphenol methoxylphenyl triazine.

22. A method of protecting a surface from ultraviolet radiation comprising topically applying to said surface a composition comprising a compound of claim I and a carrier.

23. The method of claim 22, wherein the carrier is a cosmetically acceptable carrier.

24. A method of accepting electronic singlet state excited energy from a photon-excited photoactive compound, thereby stabilizing said photoactive compound, comprising mixing said photoactive compound with a compound of formula (I) 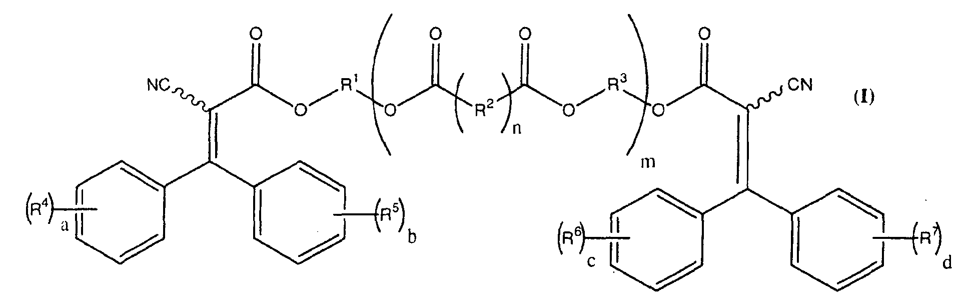 wherein R¹, R², and R³ are the same or different and are selected from the group consisting of C₁-C₃₀ alkyl, C₃-C₈ cycloalkyl, substituted alkyl, substituted cycloalkyl, ester, aryl, heteroaryl, heterocycloalkyl, substituted aryl, substituted heteroaryl, substituted heterocycloalkyl, and amino; R⁴, R⁵, R⁶, and R⁷ are each independently selected from the group consisting of hydrogen and C₁-C₃₀alkoxy, wherein R⁴ and R⁵ cannot both be hydrogen and R⁶ and R⁷ cannot both be hydrogen; a, b, c, and d are each independently 0 or 1; n is an integer of 1 to 100; and m is an integer of 0 to 100, and exposing the mixture to UV radiation in an amount sufficient for the photoactive compound to reach an electronic singlet excited state, whereby the compound of formula (I) accepts the singlet excited state energy from the excited photoactive compound, thereby returning the photoactive compound to its ground state so that it is capable of absorbing additional UV radiation.

25. The method of claim 24, wherein a, b, c, and d are each 1.

26. The method of claim 24, wherein R⁴, R⁵, R⁶, and R⁷ are each independently selected from the group consisting of hydrogen and C₁-C₈ alkoxy.

27. The method of claim 24, wherein R⁴, R⁵, R⁶, and R⁷ are each independently selected from the group consisting of hydrogen and methoxy.

28. The method of claim 27, wherein each methoxy is in the para position.

29. The method of claim 24, wherein R¹ and R³ are each neopentyl.

30. The method of claim 24, wherein R² is butyl.

31. The method of claim 24, wherein the compound of formula (I) has the structure: 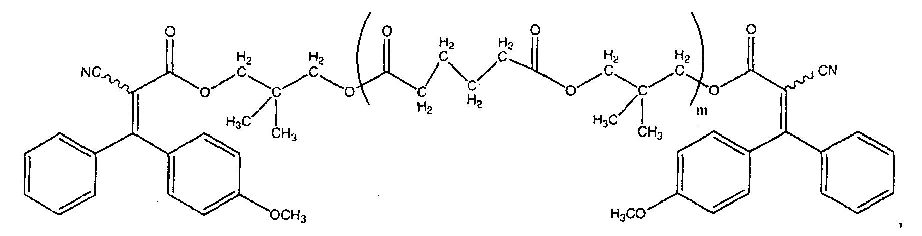 wherein m is an integer of 4 to 8.

32. The method of claim 24, wherein the compound of formula (I) is present in an amount in the weight range of 0.1 % to 20%, based on the total weight of the composition.

33. The method of claim 24, wherein the photoactive compound is selected from the group consisting of p-aminobenzoic acid and salts and derivatives thereof; anthranilate and derivatives thereof; dibenzoylmethane and derivatives thereof; salicylate and derivatives thereof; cinnamic acid and derivatives thereof; dihydroxycinnamic acid and derivatives thereof; camphor and salts and derivatives thereof; trihydroxycinnamic acid and derivatives thereof; dibenzalacetone naptholsulfonate and salts and derivatives thereof; bebzalacetophenone naphtholsulfonate and salts and derivatives thereof; dihydroxy-naphthoic acid and salts thereof; o-hydroxydiphenyldisulfonate and salts and derivatives thereof; p-hydroxdydiphenyldisulfonate and salts and derivatives thereof; coumarin and derivatives thereof; diazole derivatives; quinine derivatives and salts thereof; quinoline derivatives; hydroxyl-substituted benzophenone derivatives; methoxy-substituted benzophenone derivatives; uric acid derivatives; vilouric acid derivatives; tannic acid and derivatives thereof; hydroquinone; benzophenone derivatives; 1, 3, 5- triazine derivatives; phenyldibenzimidazole tetrasulfolnate and salts and derivatives thereof; terephthalyidene dicamphor sulfonic acid and salts and derivatives thereof; methylene bis-benzotriazolyl tetramethylbutylphenol and salts and derivatives thereof; bis-ethylhexyloxyphenol methoxyphenyl triazine and salts and derivatives thereof; and combinations thereof.

34. The method of claim 33, wherein the photoactive compound is selected from the group consisting of a dibenzoylmethane derivative; a derivative of cinnamic acid; and a combination thereof.

35. The method of claim 34, wherein the photoactive compound comprises a dibenzoylmethane derivative selected from the group consisting of 2-methyldibenzoylmethane; 4- methyldibenzoylmethane; 4-isopropyldibenzoylmethane; 4-tert-butyldibenzoylmethane; 2,4-dimethydibenzoylmethane; 2-5- dimethydibenzoylmethane; 4,4-diispropyldibenzoylmethane; 4,4-dimethoxydibenzoylmethane; 4-tert-butyl-4-methoxdibenzoylmethane; 2-methyl-5-isopropy-4-methoxydibenzoylmethane; 2-methyl-5-tert-butyl-4-methoxydibenzoylmethane; 2,4-dimethyl-4-methoxydibenzoymethane; 2,6-dimethyl-4-tert-butyl-4-methoxydibenzolmthane, and combinations thereof.

36. The method of claim 34, wherein the photoactive compound comprises 2-ethylhexyl-p-methoxycinnamate.

## Patentansprüche

1. Verbindung der Formel (I): 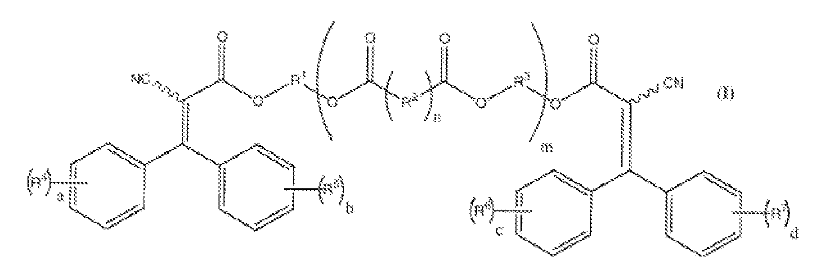 in der R¹, R² und R³ gleich oder verschieden sind und aus der Gruppe bestehend aus C₁-C₃₀-Alkyl, C₃-C₈-Cycloalkyl, substituiertes Alkyl, substituiertes Cycloaklyl, Ester, Aryl, Heteroaryl, Heterocycloaryl, substituiertes Aryl, substituiertes Heteroaryl, substituiertes Heterocycloalkyl, und Amino ausgewählt sind; R⁴, R⁵, R⁶ und R⁷ jeweils unabhängig voneinander aus der Gruppe bestehend aus Wasserstoff und C₁-C₃₀-Alkoxy ausgewählt sind, wobei R⁴ und R⁵ nicht beide Wasserstoff sein können, und R⁶ und R⁷ nicht beide Wasserstoff sein können; a, b, c und d jeweils unabhängig 0 oder 1 sind; n eine ganze Zahl von 1 bis 100 ist, und m eine ganze Zahl von 1 bis 100 ist.

2. Verbindung nach Anspruch 1, bei der a, b, c und d jeweils 1 sind.

3. Verbindung nach Anspruch 1, bei der R⁴, R⁵, R⁶ und R⁷ jeweils unabhängig aus der Gruppe bestehend aus Wasserstoff und C₁-C₈-Alkoxy ausgewählt sind.

4. Verbindung nach Anspruch 3, bei der R⁴, R⁵, R⁶ und R⁷ jeweils unabhängig aus der Gruppe bestehend aus Wasserstoff und Methoxy ausgewählt sind.

5. Verbindung nach Anspruch 4, bei der sich jedes Methoxy in der para-Position befindet.

6. Verbindung nach Anspruch 1, bei der R¹ und R³ jeweils Neopentyl sind.

7. Verbindung nach Anspruch 1, bei der R² Butyl ist.

8. Verbindung nach Anspruch 1 mit der Formel: 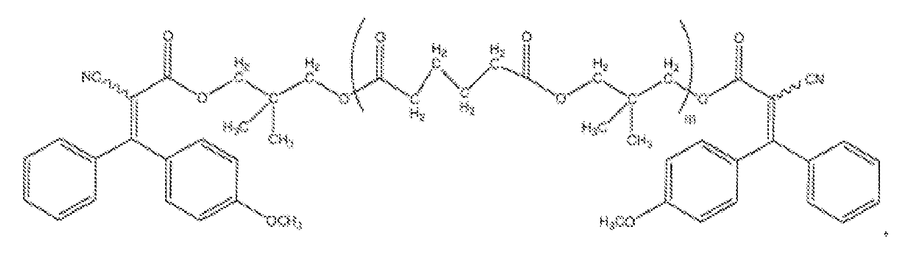 in der m eine ganze Zahl von 4 bis 8 ist.

9. Zusammensetzung, die eine Verbindung gemäß Anspruch 1 enthält.

10. Verbindung nach Anspruch 9, die weiterhin ein Additiv umfasst, das aus der Gruppe bestehend aus kosmetisch verträglichen Weichmachern, Stabilisatoren, Emulgierungsmitteln, Verdickern, Feuchthaltemitteln, Tensiden, Konservierungsmitteln, Vitaminen, Antischaummitteln, Duftstoffen, Antireizmitteln, organo-modifizerten Silikonen, Chelatisierungsmitteln, Opafizierungsmitteln, polaren Ölen, nicht polaren Ölen, Wachsen, Alkoholen, Polyolen, Treibmitteln, Farbstoffen, Pigmenten und Kombinationen davon ausgewählt ist.

11. Zusammensetzung nach Anspruch 9, die weiterhin ein Dibenzoylmethan-Derivat umfasst.

12. Zusammensetzung nach Anspruch 11, bei der das Dibenzoyl-Derivat aus der Gruppe bestehend aus 2-Methyldibenzoylmethan, 4-Methyldibenzoylmethan, 4-Isopropyldibenzoylmethan, 4-tert-Butyldibenzoylmethan, 2,4-Dimethyldibenzoylmethan, 2,5-Dimethyldibenzoylmethan, 4,4'-Diisopropyldibenzoylmethan, 4,4'-Dimethoxydibenzoylmethan, 4-tert-Butyl-4'-methoxydibenzoylmethan, 2-Methyl-5-Isopropyl-4'-methoxydibenzoylmethan, 2-Methyl-5-tert-butyl-4'-methoxydibenzoylmethan, 2,4-Dimethyl-4'-methoxydibenzoylmethan, 2,6-Dimethyl-4-tert-butyl-4'-methoxydibenzoylmethan und Kombinationen davon ausgewählt ist.

13. Zusammensetzung nach Anspruch 9, die weiterhin eine photoaktive Verbindung umfasst, die aus der Gruppe bestehend aus p-Aminobenzoesäure und Salzen und Derivaten davon; Anthranilat und Derivaten davon; Salicylat und Derivaten davon; Zimtsäure und Derivaten davon; Dihydroxyzimtsäure und Derivaten davon; Kampfer und Salzen und Derivaten davon; Trihydroxyzimtsäure und Derivaten davon; Dibenzalaceton-Naphtholsulfonat und Salzen und Derivaten davon; Benzalacetophenon-Naphtholsulfonat und Salzen und Derivaten davon; Dihydroxy-naphthlalinäure und Salzen davon; Naphthalin-Dicarbonsären, Derivaten, Dimeren, Oligimeren, Polymeren und Salzen und Kombinationen davon; o-Hydroxydiphenyldisulfonat und Salzen und Derivaten davon; p-Hydroxydiphenyldisulfonat und Salzen und Derivaten davon; Cumarin und Derivaten davon; Diazolderivate; Chininderivaten und Salzen davon; Chinolinderivaten; hydroxysubstitutierten Benzophenonderivaten; methoxysubstitutierten Benzophenonederivaten; Ureasäurederivaten; Viluronsärederivaten; Tanninsäure und Derivaten davon; Hydrochinon; Benzophenonderivaten; 1,3,5-Triazinderivaten; Phenyldibenzimidazoltetrasulfonat und Salzen und Derivaten davon; Terephthalyliden-dicampfersulfonsäure und Salzen und Derivaten davon; Methylene-bisbenzotriazolyltetramethylbutylphenol und Salzen und Derivaten davon; bis-Ethylhexyloxyphenolmethoxyphenyltriazin und Salzen und Derivaten davon; Diethylaminohydroxybenzoylhexylbenzoat und Salzen und Derivaten davon; und Kombinationen davon ausgewählt ist.

14. Verfahren zur Stabilisierung einer photozersetzbaren UV-absorbierenden Verbindung oder eines photozersetzbaren UV-absorbierenden Polymers, bei dem eine photostabilisierende Menge einer Verbindung gemäß Anspruch 1 zu der photozersetzbaren UV-absorbierenden Verbindung oder dem photozersetzbaren UV-absorbierenden Polymer gegeben wird.

15. Verfahren nach Anspruch 14, bei dem die photozersetzbare UV-absorbierende Verbindung oder das photozersetzbare UV-absorbierende Polymer ein Dibenzoylmethanderivat ist, das aus der Gruppe bestehend aus 2-Methyldibenzoylmethan, 4-Methyldibenzoylmethan, 4-Isopropyldibenzoylmethane, 4-tert-butyldibenzoylmethan, 2,4-Dimethyldibenzoylmethan, 2,5-Dimethyldibenzoylmethan, 4,4'-Diisopropyldibenzoylmethan, 4,4'-Dimethoxydibenzoylmethan, 4-tert-Butyl-4'-methoxydibenzoylmethan, 2-Methyl-5-isopropyl-4'-methoxydibenzoylmethan, 2-Methyl-5-tert-butyl-4'-methoxydibenzoylmethan, 2,4-Dimethyl-4'-methoxydibenzoylmethan, 2,6-Dimethyl-4-tert-butyl-4'-methoxydibenzoylmethan und Kombinationen davon ausgewählt ist.

16. Verfahren nach Anspruch 15, bei dem weiterhin ein Tripletfänger zugegeben wird, der aus der Gruppe bestehend aus Octocrylene, Methylbenzylidenekampfer, Diethylhexyl-2,6-naphthalat, Diethylhexylsyringylidenmalonat und Kombinationen davon ausgewählt ist.

17. Verfahren nach Anspruch 15, bei dem weiterhin Benzophenon-3 zugegeben wird.

18. Verfahren nach Anspruch 15, bei dem weiterhin Octylsalicylat zugegeben wird.

19. Verfahren nach Anspruch 15, bei dem weiterhin Cinnamatester zugegeben wird.

20. Verfahren nach Anspruch 19, bei dem der Cinnamatester 2-Ethylhexyl-p-methoxycinnamat ist.

21. Verfahren nach Anspruch 19, bei dem weiterhin Bis-ethylhexyloxyphenol-methoxyphenyltriazin zugegeben wird.

22. Verfahren zum Schützen einer Oberfläche gegen ultraviolette Strahlung, bei der eine Zusammensetzung topisch auf eine Oberfläche aufgetragen wird, die eine Verbindung gemäß Anspruch 1 und einen Träger umfasst.

23. Verfahren nach Anspruch 22, bei der der Träger ein kosmetisch verträglicher Träger ist.

24. Verfahren zur Annahme von durch elektronischen Singletzustand angeregte Energie aus einer durch Photonen angeregten photoaktiven Verbindung, wobei die photoaktive Verbindung stabilisiert wird, bei dem die photoaktive Verbindung mit einer Verbindung der Formel (I) gemischt wird 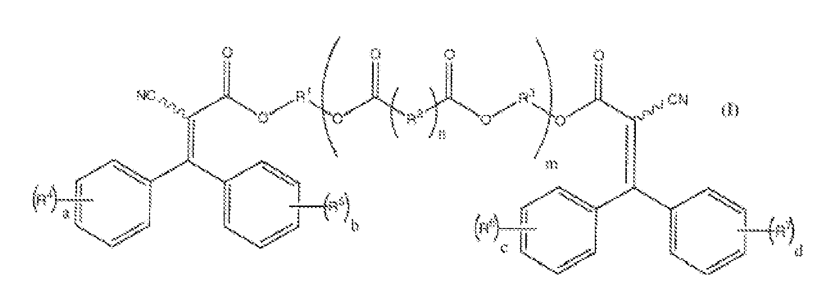 in der R¹, R² und R³ gleich oder verschieden sind und aus der Gruppe bestehend aus C₁-C₃₀-Alkyl, C₃-C₈-Cycloalkyl, substituiertes Alkyl, substituiertes Cycloaklyl, Ester, Aryl, Heteroaryl, Heterocycloaryl, substituiertes Aryl, substituiertes Heteroaryl, substituiertes Heterocycloalkyl, und Amino ausgewählt sind; R⁴, R⁵, R⁶ und R⁷ jeweils unabhängig voneinander aus der Gruppe bestehend aus Wasserstoff und C₁-C₃₀-Alkoxy ausgewählt sind, wobei R⁴ und R⁵ nicht beide Wasserstoff sein können, und R⁶ und R⁷ nicht beide Wasserstoff sein können; a, b, c und d jeweils unabhängig 0 oder 1 sind; n eine ganze Zahl von 1 bis 100 ist, und m eine ganze Zahl von 1 bis 100 ist, und die Mischung in einer ausreichenden Menge UV-Strahlung ausgesetzt wird, so dass die photoaktive Verbindung einen elektronischen angeregten Singletzustand erreicht, wobei die Verbindung der Formel (I) die durch elektronischen Singletzustand angeregte Energie aus der angeregten photoaktiven Verbindung aufnimmt, wodurch die photoaktive Verbindung in ihren Grundzustand zurückkehrt, so dass sie zusätzliche UV-Strahlung absorbieren kann.

25. Verfahren nach Anspruch 24, bei dem a, b, c und d jeweils 1 sind.

26. Verfahren nach Anspruch 24, bei dem R⁴, R⁵, R⁶ und R⁷ jeweils unabhängig aus der Gruppe bestehend aus Wasserstoff und C₁-C₈-Alkoxy ausgewählt sind.

27. Verfahren nach Anspruch 24, bei dem R⁴, R⁵, R⁶ und R⁷ jeweils unabhängig aus der Gruppe bestehend aus Wasserstoff und Methoxy ausgewählt sind.

28. Verfahren nach Anspruch 27, bei dem sich jedes Methoxy in der para-Position befindet.

29. Verfahren nach Anspruch 24, bei dem R¹ und R³ jeweils Neopentyl sind.

30. Verfahren nach Anspruch 24, bei dem R² Butyl ist.

31. Verfahren nach Anspruch 24, bei dem die Verbindung mit der Formel (I) folgende Struktur aufweist: 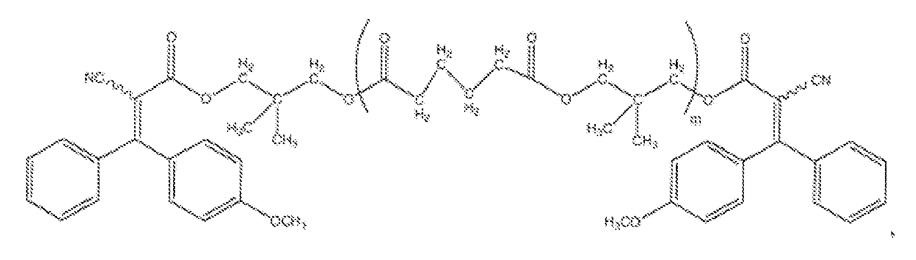 in der m eine ganze Zahl von 4 bis 8 ist.

32. Verfahren nach Anspruch 24, bei dem die Verbindung der Formel (I) in einer Gewichtsmenge im Bereich von 0,1% bis 20% auf Basis des Gesamtgewichts der Zusammensetzung vorhanden ist.

33. Verfahren nach Anspruch 24, bei dem die photoaktive Verbindung aus der Gruppe bestehend aus p-Aminobenzoesäure und Salzen und Derivaten davon; Anthranilat und Derivaten davon; Salicylat und Derivaten davon; Zimtsäure und Derivaten davon; Dihydroxyzimtsäure und Derivaten davon; Kampfer und Salzen und Derivaten davon; Trihydroxyzimtsäure und Derivaten davon; Dibenzalaceton- Naphtholsulfonat und Salzen und Derivaten davon; Benzalacetophenon-Naphtholsulfonat und Salzen und Derivaten davon; Dihydroxy-naphthlalinäure und Salzen davon; Naphthalin-Dicarbonsären, Derivaten, Dimeren, Oligimeren, Polymeren und Salzen und Kombinationen davon; o-Hydroxydiphenyldisulfonat und Salzen und Derivaten davon; p-Hydroxydiphenyldisulfonat und Salzen und Derivaten davon; Cumarin und Derivaten davon; Diazolderivate; Chininderivaten und Salzen davon; Chinolinderivaten; hydroxysubstitutierten Benzophenonderivaten; methoxysubstitutierten Benzophenonederivaten; Ureasäurederivaten; Viluronsärederivaten; Tanninsäure und Derivaten davon; Hydrochinon; Benzophenonderivaten; 1,3,5-Triazinderivaten; Phenyldibenzimidazoltetrasulfonat und Salzen und Derivaten davon; Terephthalyliden-dicampfersulfonsäure und Salzen und Derivaten davon; Methylene-bisbenzotriazolyltetramethylbutylphenol und Salzen und Derivaten davon; bis-Ethylhexyloxyphenolmethoxyphenyltriazin und Salzen und Derivaten davon; Diethylaminohydroxybenzoylhexylbenzoat und Salzen und Derivaten davon; und Kombinationen davon ausgewählt ist.

34. Verfahren nach Anspruch 33, bei dem die photoaktive Verbindung aus der Gruppe bestehend aus Dibenoylmethanderivat, Zimtsäurederivat und einer Kombination davon ausgewählt ist.

35. Verfahren nach Anspruch 34, die photoaktive Verbindung ein Dibenzoyl-Derivat umfasst, das aus der Gruppe bestehend aus 2-Methyldibenzoylmethan, 4-Methyldibenzoylmethan, 4-Isopropyldibenzoylmethan, 4-tert-Butyldibenzoylmethan, 2,4-Dimethyldibenzoylmethan, 2,5-Dimethyldibenzoylmethan, 4,4'-Diisopropyldibenzoylmethan, 4,4'-Dimethoxydibenzoylmethan, 4-tert-Butyl-4'-methoxydibenzoylmethan, 2-Methyl-5-isopropyl-4'-methoxydibenzoylmethan, 2-Methyl-5-tert-butyl-4'-methoxydibenzoylmethan, 2,4-Dimethyl-4'-methoxydibenzoylmethan, 2,6-Dimethyl-4-tert-butyl-4'-methoxydibenzoylmethan und Kombinationen davon ausgewählt ist.

36. Verfahren nach Anspruch 34, bei dem die photoaktive Verbindung 2-Ethylhexyl-p-methoxycinnamat umfasst.

## Revendications

1. Composé de formule (I) : 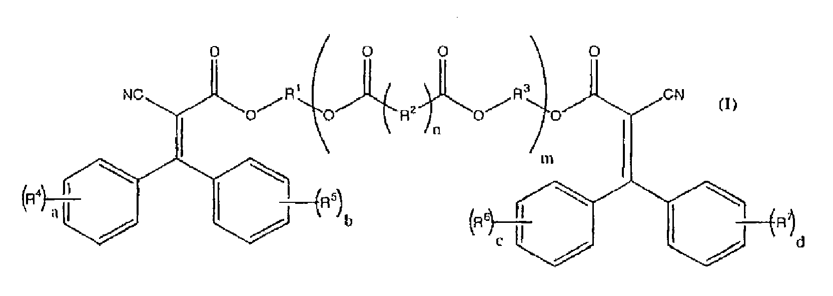 où R¹, R² et R³ sont identiques ou différents et sont choisis dans le groupe consistant en C₁-C₃₀ alkyle, C₃-C₈ cycloalkyle, alkyle substitué, cycloalkyle substitué, ester, aryle, hétéroaryle, hétérocycloalkyle, aryle substitué, hétéroaryle substitué, hétérocycloalkyle substitué, et amino ; R⁴, R⁵, R⁶ et R⁷ sont choisis chacun indépendamment dans le groupe consistant en hydrogène et C₁-C₃₀ alcoxy, où R⁴ et R⁵ ne peuvent pas être l'un et l'autre hydrogène et R⁶ et R⁷ ne peuvent pas être l'un et l'autre hydrogène ; a, b, c et d sont chacun indépendamment 0 ou 1 ; n est un entier de 1 à 100 ; et m est un entier de 0 à 100.

2. Composé selon la revendication 1 où a, b, c et d sont chacun 1.

3. Composé selon la revendication 1 où R⁴, R⁵ R⁶ et R⁷ sont choisis chacun indépendamment dans le groupe consistant en hydrogène et C₁-C₈ alcoxy.

4. Composé selon la revendication 3 où R⁴, R⁵ R⁶ et R⁷ sont choisis chacun indépendamment dans le groupe consistant en hydrogène et méthoxy.

5. Composé selon la revendication 4 où chaque méthoxy est dans la position para.

6. Composé selon la revendication 1 où R¹ et R³ sont chacun néopentyle.

7. Composé selon la revendication 1 où R² est butyle.

8. Composé selon la revendication 1, ayant la formule : 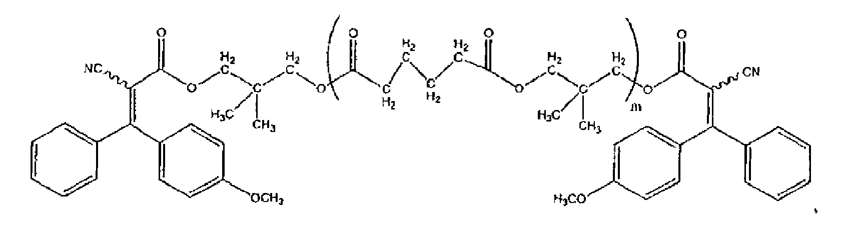 où m est un entier de 4 à 8.

9. Composition comprenant un composé selon la revendication 1.

10. Composition selon la revendication 9 comprenant en outre un additif choisi dans le groupe consistant en les émollients, stabilisants, émulsifiants, épaississants, humectants, tensioactifs, conservateurs, vitamines, agents antimousse, parfums, anti-irritants, silicones organo-modifiés, chélateurs, opacifiants, huiles polaires, huiles non polaires, cires, alcools, polyols, propulseurs, colorants, pigments acceptables du point de vue cosmétique, et leurs combinaisons.

11. Composition selon la revendication 9 comprenant en outre un dérivé de dibenzoylméthane.

12. Composition selon la revendication 11 où ledit dérivé de dibenzoylméthane est choisi dans le groupe consistant en 2-méthyldibenzoylméthane ; 4-méthyldibenzoylméthane ; 4-isopropyldibenzoylméthane ; 4-tert-butyldibenzoylméthane ; 2,4-diméthyldibenzoylméthane ; 2,5-diméthyldibenzoylméthane ; 4,4'-diisopropyldibenzoylméthane ; 4,4'-diméthoxydibenzoylméthane ; 4-tert-butyl-4'-méthoxydibenzoylméthane ; 2-méthyl-5-isopropyl-4'-méthoxydibenzoylméthane ; 2-méthyl-5-tert-butyl-4'-méthoxydibenzoylméthane ; 2,4-diméthyl-4'-méthoxydibenzoylméthane ; 2,6-diméthyl-4-tert-butyl-4'-méthoxydibenzoylméthane ; et leurs combinaisons.

13. Composition selon la revendication 9 comprenant en outre un composé photoactif choisi dans le groupe consistant en l'acide p-aminobenzoïque et ses sels et dérivés ; un anthranilate et ses dérivés ; un salicylate et ses dérivés ; l'acide cinnamique et ses dérivés ; l'acide dihydroxycinnamique et ses dérivés ; le camphre et ses sels et dérivés ; l'acide trihydroxycinnamique et ses dérivés ; le dibenzalacétone naphtolsulfonate et ses sels et dérivés ; le benzalacétophénone naphtolsulfonate et ses sels et dérivés ; l'acide dihydroxynaphtoïque et ses sels ; les acides naphtalènedicarboxyliques, leurs dérivés, dimères, oligomères, polymères, et leurs sels et combinaisons ; le o-hydroxydiphényldisulfonate et ses sels et dérivés ; le p-hydroxydiphényldisulfonate et ses sels et dérivés ; la coumarine et ses dérivés ; les dérivés du diazole ; les dérivés de la quinine et leurs sels ; les dérivés de la quinoléine ; les dérivés de benzophénone hydroxy-substitués ; les dérivés de benzophénone méthoxy-substitués ; les dérivés de l'acide urique ; les dérivés de l'acide vilourique ; l'acide tannique et ses dérivés ; l'hydroquinone ; les dérivés de benzophénone ; les dérivés de 1,3,5-triazine, le phényldibenzimidazole tétrasulfonate et ses sels et dérivés ; l'acide téréphtalylidène dicamphosulfonique et ses sels et dérivés ; le méthylène bis-benzotriazolyl tétraméthylbutylphénol et ses sels et dérivés; la bis-éthylhexyloxyphénol méthoxyphényl triazine et ses sels et dérivés ; le diéthylamino hydroxybenzoyl hexyl benzoate et ses sels et dérivés ; et leurs combinaisons.

14. Procédé pour photostabiliser un composé ou polymère absorbant les UV photodégradable comprenant l'addition d'une quantité photostabilisante d'un composé selon la revendication 1 au composé ou polymère absorbant les UV photodégradable.

15. Procédé selon la revendication 14 où le composé ou polymère absorbant les UV photodégradable est un dérivé de dibenzoylméthane est choisi dans le groupe consistant en 2-méthyldibenzoylméthane ; 4-méthyldibenzoylméthane ; 4-isopropyldibenzoylméthane ; 4-tert-butyldibenzoylméthane ; 2,4-diméthyldibenzoylméthane ; 2,5-diméthyldibenzoylméthane ; 4,4'-diisopropyldibenzoylméthane ; 4,4'-diméthoxydibenzoylméthane ; 4-tert-butyl-4'-méthoxydibenzoylméthane ; 2-méthyl-5-isopropyl-4'-méthoxydibenzoylméthane ; 2-méthyl-5-tert-butyl-4'-méthoxydibenzoylméthane ; 2,4-diméthyl-4'-méthoxydibenzoylméthane ; 2,6-diméthyl-4-tert-butyl-4'-méthoxydibenzoylméthane ; et leurs combinaisons.

16. Procédé selon la revendication 15 comprenant en outre l'addition d'un extincteur de triplet choisi dans le groupe consistant en octocrylène, méthyl benzylidène camphre, 2,6-naphtalate de diéthylhexyle, syringylidène malonate de diéthylhexyle, et leurs combinaisons.

17. Procédé selon la revendication 15 comprenant en outre l'addition de benzophénone-3.

18. Procédé selon la revendication 15 comprenant en outre l'addition de salicylate d' octyle.

19. Procédé selon la revendication 15 comprenant en outre l'addition d'un ester cinnamate.

20. Procédé selon la revendication 19 où l'ester cinnamate est le p-méthoxycinnamate de 2-éthylhexyle.

21. Procédé selon la revendication 19 comprenant en outre l'addition de bis-éthylhexyloxyphénol méthoxyphényl triazine.

22. Procédé de protection d'une surface contre les rayonnements ultraviolets comprenant l'application topique à ladite surface d'une composition comprenant un composé selon la revendication 1 et un vecteur.

23. Procédé selon la revendication 22 où le vecteur est un vecteur acceptable du point de vue cosmétique.

24. Procédé pour accepter l'énergie de l'état excité de singulet électronique d'un composé photoactif excité par des photons, pour stabiliser ainsi ledit composé photoactif, comprenant le mélange dudit composé photoactif avec un composé de formule (I) 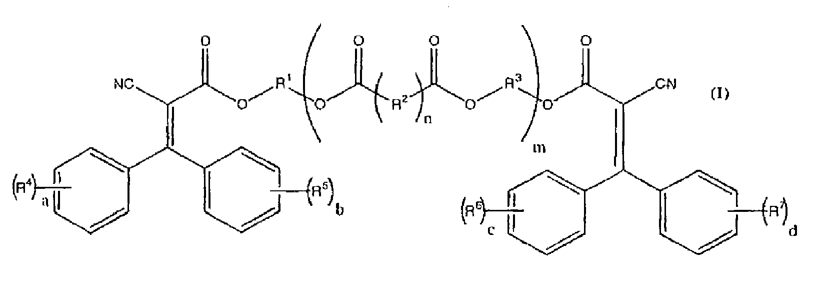 où R¹, R² et R³ sont identiques ou différents et sont choisis dans le groupe consistant en C₁-C₃₀ alkyle, C₃-C₈ cycloalkyle, alkyle substitué, cycloalkyle substitué, ester, aryle, hétéroaryle, hétérocycloalkyle, aryle substitué, hétéroaryle substitué, hétérocycloalkyle substitué et amino ; R⁴ R⁵ R⁶ et R⁷ sont choisis chacun indépendamment dans le groupe consistant en hydrogène et C₁-C₃₀ alcoxy, où R⁴ et R⁵ ne peuvent pas être l'un et l'autre hydrogène et R⁶ et R⁷ ne peuvent pas être l'un et l'autre hydrogène ; a, b, c et d sont chacun indépendamment 0 ou 1 ; n est un entier de 1 à 100 ; et m est un entier de 0 à 100, et l'exposition du mélange à un rayonnement UV en une quantité suffisante pour que le composé photoactif atteigne un état excité de singulet électronique, de sorte que le composé de formule (I) accepte l'énergie de l'état excité de singulet provenant du composé photoactif excité, en ramenant ainsi le composé photoactif à son état fondamental de sorte qu'il est capable d'absorber un rayonnement UV supplémentaire.

25. Procédé selon la revendication 24 où a, b, c et d sont chacun 1.

26. Procédé selon la revendication 24 où R⁴, R⁵, R⁶ et R⁷ sont choisis chacun indépendamment dans le groupe consistant en hydrogène et C₁-C₈ alcoxy.

27. Procédé selon la revendication 24 où R⁴, R⁵, R⁶ et R⁷ sont choisis chacun indépendamment dans le groupe consistant en hydrogène et méthoxy.

28. Procédé selon la revendication 27 où chaque méthoxy est dans la position para.

29. Procédé selon la revendication 24 où R¹ et R³ sont chacun néopentyle.

30. Procédé selon la revendication 24 où R² est butyle.

31. Procédé selon la revendication 24 où le composé de formule (I) a la structure : 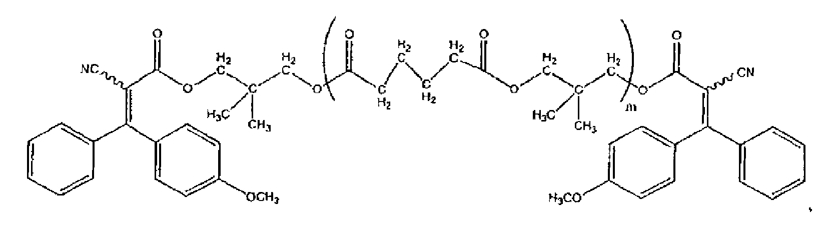 où m est un entier de 4 à 8.

32. Procédé selon la revendication 24 où le composé de formule (I) est présent en une quantité dans la plage en poids de 0,1 % à 20 %, sur la base du poids total de la composition.

33. Procédé selon la revendication 24 où le composé photoactif est choisi dans le groupe consistant en l'acide p-aminobenzoïque et ses sels et dérivés ; un anthranilate et ses dérivés ; le dibenzoylméthane et ses dérivés ; un salicylate et ses dérivés ; l'acide cinnamique et ses dérivés ; l'acide dihydroxycinnamique et ses dérivés ; le camphre et ses sels et dérivés ; l'acide trihydroxycinnamique et ses dérivés ; le dibenzalacétone naphtolsulfonate et ses sels et dérivés ; le benzalacétophénone naphtolsulfonate et ses sels et dérivés ; l'acide dihydroxynaphtoïque et ses sels ; le o-hydroxydiphényldisulfonate et ses sels et dérivés ; le p-hydroxydiphényl-disulfonate et ses sels et dérivés ; la coumarine et ses dérivés ; les dérivés du diazole ; les dérivés de la quinine et leurs sels ; les dérivés de la quinoléine ; les dérivés de benzophénone hydroxy-substitués ; les dérivés de benzophénone méthoxy-substitués ; les dérivés de l'acide urique ; les dérivés de l'acide vilourique ; l'acide tannique et ses dérivés ; l'hydroquinone ; les dérivés de benzophénone ; les dérivés de 1,3,5-triazine ; le phényldibenzimidazole tétrasulfonate et ses sels et dérivés ; l'acide téréphtalylidène dicamphosulfonique et ses sels et dérivés ; le méthylène bis-benzotriazolyl tétraméthylbutylphénol et ses sels et dérivés ; la bis-éthylhexyloxyphénol méthoxyphényl triazine et ses sels et dérivés ; et leurs combinaisons.

34. Procédé selon la revendication 33 où le composé photoactif est choisi dans le groupe consistant en un dérivé du dibenzoylméthane ; un dérivé de l'acide cinnamique ; et une combinaison de ceux-ci.

35. Procédé selon la revendication 34 où le composé photoactif comprend un dérivé de dibenzoylméthane choisi dans le groupe consistant en 2-méthyldibenzoylméthane ; 4-méthyldibenzoylméthane ; 4-isopropyldibenzoylméthane ; 4-tert-butyldibenzoylméthane ; 2,4-diméthyldibenzoylméthane ; 2,5-diméthyldibenzoylméthane ; 4,4'-diisopropyldibenzoylméthane ; 4,4'-diméthoxydibenzoylméthane ; 4-tert-butyl-4'-méthoxydibenzoylméthane ; 2-méthyl-5-isopropyl-4'-méthoxydibenzoylméthane ; 2-méthyl-5-tert-butyl-4'-méthoxydibenzoylméthane ; 2,4-diméthyl-4'-méthoxydibenzoylméthane ; 2,6-diméthyl-4-tert-butyl-4'-méthoxydibenzoylméthane ; et leurs combinaisons.

36. Procédé selon la revendication 34 où le composé photoactif comprend du p-méthoxycinnamate de 2-éthylhexyle.
